# EUROPEAN PATENT SPECIFICATION

(11) **EP 4 559 904 B1**
(45) Date of publication and mention of the grant of the patent: **02.12.2026**
(21) Application number: 23854433.2
(22) Date of filing: 15.08.2023
(51) Int. Cl.: C07D 313/00, C07D 495/04, A61P 5/02

(54) **SALT TYPE AND CRYSTAL FORM OF THIENOPYRIMIDINONE DERIVATIVE**
SALZTYP UND KRISTALLFORM EINES THIENOPYRIMIDINONDERIVATS
FORME SALINE ET FORME CRISTALLINE DE DÉRIVÉ DE THIÉNOPYRIMIDINONE

(30) Priority: 16.08.2022 CN 202210984344
(43) Date of publication of application: 28.05.2025
(73) Proprietor: CMS Research & Development Pte. Ltd., Singapore 179803 (SG)
(72) Inventor: HU, Boyu, Shanghai 200131 (CN); CHEN, Kevin X, Shanghai 200131 (CN); LIU, Bin, Shanghai 200131 (CN)
(74) Representative: Maiwald GmbH
(86) International application number: PCT/CN2023/113085
(87) International publication number: WO 2024/037532

(56) References cited:
- EP-A1- 3 498 280
- WO-A1-2022/179476
- WO-A2-2020/094698
- CN-A- 105 793 267
- CN-A- 114 364 384
- CN-A- 114 466 665
- US-A1- 2009 325 900

## Description

This application claims the priority of:
CN202210984344.5, filed on August 16, 2022.

### TECHNICAL FIELD

The present disclosure relates to salt types and crystal forms of a thienopyrimidone derivative, and preparation methods therefor. Specifically, the present disclosure discloses salt types and crystal forms of the compound of formula (I), and preparation methods therefor.

### BACKGROUND ART

Endometriosis is the appearance of endometrial glands or stroma outside the uterus. Symptoms resulted from endometriosis include chronic pelvic pain, dysmenorrhea, infertility, etc. It is extremely difficult to cure and prone to recurrence. It is considered one of the gynecological diseases difficult to cure. The clinical medication for this disease has disadvantages such as long medication time, many side effects, and inconvenient administration. It is reported that in 2021, approximately 176 million women worldwide suffered from endometriosis.

The causes of endometriosis are very complex, and the pathogenesis of endometriosis has not yet been fully clarified. Clinical drug treatment options are either to control the level of estrogen, or to control inflammation, or both. Drugs used for the treatment are mainly divided into non-steroidal anti-inflammatory drugs, progestins, combined oral contraceptives, gonadotropin-releasing hormone (GnRH) agonists, etc. Among them, the oral contraceptives have a non-response rate of nearly one-third to one-quarter in patients. The progestins have a side effect of causing obesity, and particularly, are forbidden for patients who want to get pregnant. The gonadotropin-releasing hormones have perimenopausal side effects. There are many problems with the polypeptide GnRH receptor agonists or antagonist compounds, such as the problems of oral absorbability, dosage form, dosage volume, drug stability, sustained action and metabolic stability.

GnRH receptor antagonists competitively bind to GnRH receptors, blocking the binding of GnRH to the receptors, directly inhibiting the hypothalamus-pituitary-ovarian axis, and then inhibiting the secretion of follicle-stimulating hormone and luteinizing hormone, thereby reducing the level of estrogen, with rapid onset of action and few side effects. Currently, in addition to the first-to-market Elagolix, the small molecule GnRH receptor antagonists that are at the forefront of research and development include the second small molecule oral antagonist, Relugolix, which was approved by FDA in December 2020 for the treatment of its first indication of advanced prostate cancer, while treatment of the indication of uterine fibroids has been approved in Japan, and treatment of endometriosis has entered Phase III clinical trials. The third antagonist, Linzagolix, is already in the Phase III clinical trials for the treatment of both indications of endometriosis and uterine fibroids.

The crystal structure of an active ingredient for medicinal use often affects the stability of the compound. Generally speaking, amorphous drug products do not have a regular crystal structure and often have defects, such as poor stability, easy agglomeration, small particles, and difficulty in filtration. In addition, the solubility of drug molecules is closely related to the degree of oral absorption. The solubility of drugs can be increased by forming salts with suitable acids or bases. At the same time, the stability of easily oxidized drug molecules can also be helped to increase through salt formation, which is beneficial for storage and transportation. Therefore, we need to conduct in-depth research and find suitable salt types and crystal forms of the drug molecules, particularly new crystal forms with good stability.

### SUMMARY

The present disclosure provides a crystal form B of the compound of formula (I),

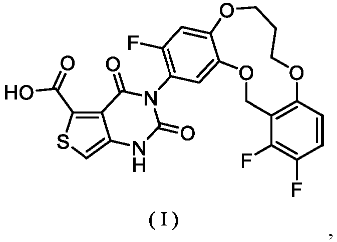

wherein the crystal form B has an X-ray powder diffraction pattern obtained using Cu Kα radiation with characteristic diffraction peaks at 2θ angles of 4.62±0.20°, 7.35±0.20°, and 18.34±0.20°.

In some embodiments of the present disclosure, the above crystal form B has an X-ray powder diffraction pattern obtained using Cu Kα radiation with characteristic diffraction peaks at 20 angles of 4.62±0.20°, 7.35±0.20°, 11.45±0.20°, 12.33±0.20°, and 18.34±0.20°.

In some embodiments of the present disclosure, the above crystal form B has an X-ray powder diffraction pattern obtained using Cu Kα radiation with characteristic diffraction peaks at 20 angles of 4.62±0.20°, 7.35±0.20°, 11.45±0.20°, 12.33±0.20°, 18.34±0.20°, 22.41±0.20°, 26.54±0.20°, and 27.08±0.20°.

In some embodiments of the present disclosure, the above crystal form B has an X-ray powder diffraction pattern obtained using Cu Kα radiation with characteristic diffraction peaks at 20 angles of 4.62±0.20°, 7.35±0.20°, 11.45±0.20°, 12.33±0.20°, 13.75±0.20°, 17.89±0.20°, 18.34±0.20°, 20.92±0.20°, 22.41±0.20°, 25.43±0.20°, 26.54±0.20°, and 27.08±0.20°.

In some embodiments of the present disclosure, the above crystal form B has an X-ray powder diffraction pattern obtained using Cu Kα radiation with characteristic diffraction peaks at 20 angles of 4.62±0.20°, 7.35±0.20°, 9.17±0.20°, 11.45±0.20°, 12.33±0.20°, 13.75±0.20°, 17.89±0.20°, 18.34±0.20°, 20.92±0.20°, 22.41±0.20°, 23.57±0.20°, 24.46±0.20°, 25.43±0.20°, 26.54±0.20°, 27.08±0.20°, and 28.81±0.20°.

In some embodiments of the present disclosure, the above crystal form B has an X-ray powder diffraction pattern obtained using Cu Kα radiation with characteristic diffraction peaks at 20 angles of 4.62±0.20°, 7.35±0.20°, 18.34±0.20°, and/or 9.17±0.20°, and/or 11.45±0.20°, and/or 12.33±0.20°, and/or 13.17±0.20°, and/or 13.75±0.20°, and/or 14.34±0.20°, and/or 14.67±0.20°, and/or 16.50±0.20°, and/or 17.43±0.20°, and/or 17.89±0.20°, and/or 19.45±0.20°, and/or 20.92±0.20°, and/or 22.41±0.20°, and/or 23.57±0.20°, and/or 24.46±0.20°, and/or 25.43±0.20°, and/or 25.89±0.20°, and/or 26.54±0.20°, and/or 27.08±0.20°, and/or 28.81±0.20°, and/or 31.69±0.20°.

In some embodiments of the present disclosure, the above crystal form B has an X-ray powder diffraction pattern obtained using Cu Kα radiation with characteristic diffraction peaks at 20 angles of 4.62°, 7.35°, 9.17°, 11.45°, 12.33°, 13.17°, 13.75°, 14.34°, 14.67°, 16.50°, 17.43°, 17.89°, 18.34°, 19.45°, 20.92°, 21.68°, 22.41°, 23.57°, 24.46°, 25.43°, 25.89°, 26.54°, 27.08°, and 28.81°.

In some embodiments of the present disclosure, the above crystal form B has an X-ray powder diffraction pattern obtained using Cu Kα radiation with characteristic diffraction peaks at 20 angles of 4.62°, 7.35°, 9.17°, 11.45°, 12.33°, 13.17°, 13.75°, 14.34°, 14.67°, 16.50°, 17.43°, 17.89°, 18.34°, 19.45°, 20.92°, 21.68°, 22.41°, 23.57°, 24.46°, 25.43°, 25.89°, 26.54°, 27.08°, 28.81°, 30.27°, 31.69°, 33.32°, 33.71°, 33.99°, 35.25°, 36.92°, and 37.69°.

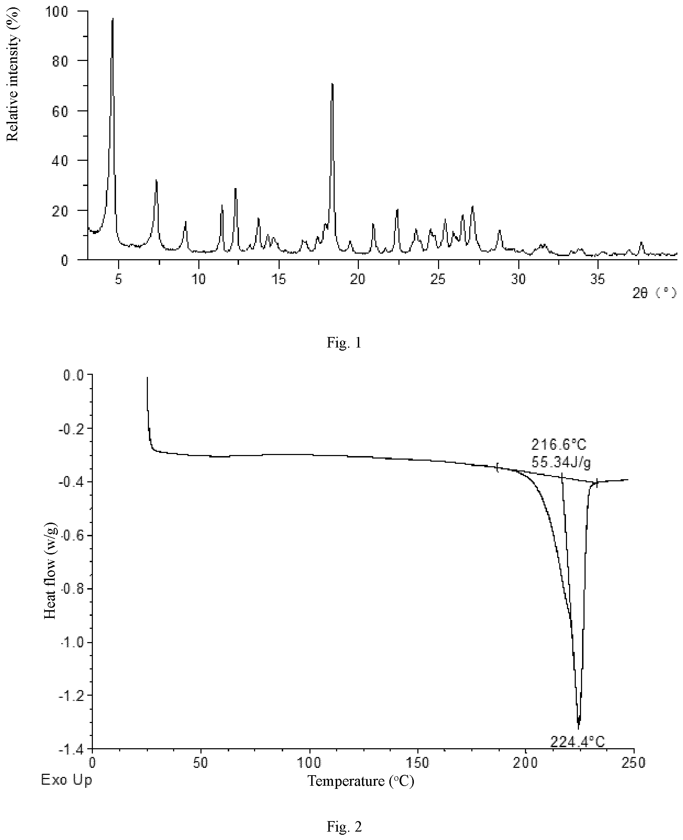

In some embodiments of the present disclosure, the XRPD pattern of the above crystal form B obtained using Cu Kα radiation is substantially as shown in Fig. 1.

In some embodiments of the present disclosure, in the XRPD pattern of the above crystal form B obtained using Cu Kα radiation, data of the diffraction peaks are as shown in Table 1:

**Table 1 XRPD data of the crystal form B of the compound of formula (I)**

| **No.** | 2θ[°] | Peak height [cts] | Left width at half maximum [°2θ] | D-spacing [Å] | Relative intensity [%] |
|---|---|---|---|---|---|
| 1 | 4.6176 | 7764.48 | 0.1791 | 19.14 | 100.00 |
| 2 | 7.3516 | 2436.15 | 0.1791 | 12.03 | 31.38 |
| 3 | 9.1718 | 968.48 | 0.1279 | 9.64 | 12.47 |
| 4 | 11.4452 | 1679.55 | 0.1279 | 7.73 | 21.63 |
| 5 | 12.3266 | 2308.77 | 0.1535 | 7.18 | 29.73 |
| 6 | 13.1713 | 258.23 | 0.1535 | 6.72 | 3.33 |
| 7 | 13.7523 | 1241.19 | 0.1535 | 6.44 | 15.99 |
| 8 | 14.3445 | 670.23 | 0.1791 | 6.17 | 8.63 |
| 9 | 14.6676 | 554.09 | 0.1279 | 6.04 | 7.14 |
| 10 | 16.4957 | 475.43 | 0.1023 | 5.37 | 6.12 |
| 11 | 17.4348 | 607.16 | 0.1535 | 5.09 | 7.82 |
| 12 | 17.8903 | 1067.77 | 0.1535 | 4.96 | 13.75 |
| 13 | 18.3367 | 6232.24 | 0.1791 | 4.84 | 80.27 |
| 14 | 19.4470 | 463.18 | 0.1791 | 4.56 | 5.97 |
| 15 | 20.9207 | 1075.81 | 0.1279 | 4.25 | 13.86 |
| 16 | 21.6806 | 207.64 | 0.2047 | 4.10 | 2.67 |
| 17 | 22.4067 | 1632.82 | 0.1791 | 3.97 | 21.03 |
| 18 | 23.5677 | 936.32 | 0.1279 | 3.78 | 12.06 |
| 19 | 24.4607 | 879.36 | 0.1791 | 3.64 | 11.33 |
| 20 | 25.4287 | 1234.01 | 0.1791 | 3.50 | 15.89 |
| 21 | 25.8940 | 788.90 | 0.1279 | 3.44 | 10.16 |
| 22 | 26.5394 | 1356.36 | 0.2047 | 3.36 | 17.47 |
| 23 | 27.0844 | 1762.08 | 0.2558 | 3.29 | 22.69 |
| 24 | 28.8123 | 911.37 | 0.2814 | 3.10 | 11.74 |
| 25 | 30.2663 | 185.91 | 0.2047 | 2.95 | 2.39 |
| 26 | 31.6866 | 377.11 | 0.1535 | 2.82 | 4.86 |
| 27 | 33.3166 | 159.07 | 0.1535 | 2.69 | 2.05 |
| 28 | 33.7081 | 236.58 | 0.1535 | 2.66 | 3.05 |
| 29 | 33.9866 | 220.96 | 0.1535 | 2.64 | 2.85 |
| 30 | 35.2528 | 119.10 | 0.3582 | 2.55 | 1.53 |
| 31 | 36.9151 | 171.26 | 0.2047 | 2.44 | 2.21 |
| 32 | 37.6890 | 467.62 | 0.1791 | 2.39 | 6.02 |

In one embodiment of the present disclosure, the differential scanning calorimetry (DSC) curve of the above crystal form B shows a peak value of an exothermic peak at 224.4 °C±3 °C.

In one embodiment of the present disclosure, the DSC curve of the above crystal form B is substantially as shown in Fig. 2.

In one embodiment of the present disclosure, the thermogravimetric analysis (TGA) curve of the above crystal form B has a weight loss of 1.12% at 150 °C±3 °C.

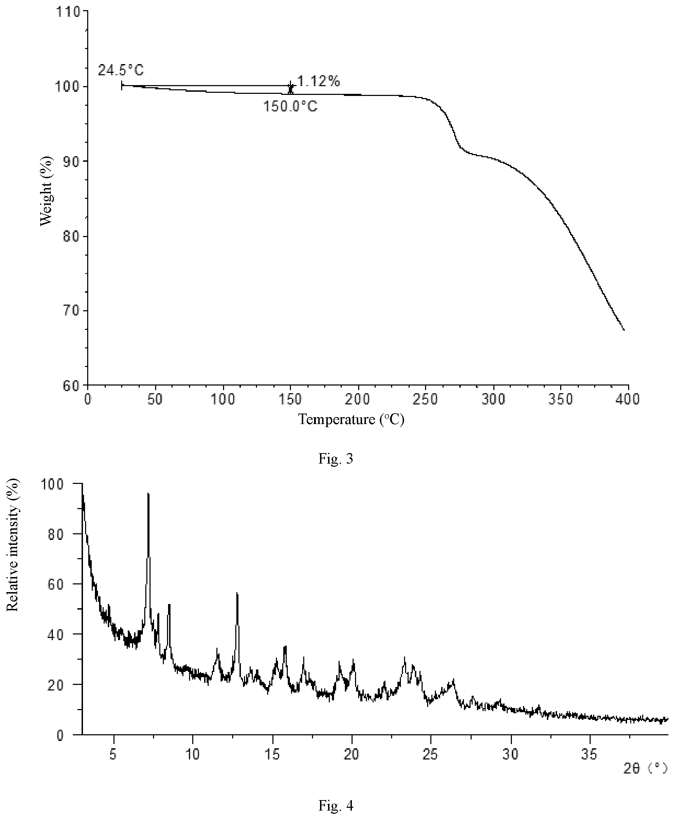

In one embodiment of the present disclosure, the TGA curve of the above crystal form B is substantially as shown in Fig. 3.

The present disclosure also provides a method of preparing the crystal form B of the compound of formula (I), comprising the following steps:
(a) adding the compound of formula (I) to water or a mixed solvent of acetonitrile and water (with a volume ratio of 1:5 to 1:50) at 70 °C to 95 °C;
(b) stirring for 10 to 48 hours;
(c) filtering;
(d) drying the filter cake at 50 °C to 70 °C.

The present disclosure also provides a crystal form C of the compound of formula (I), wherein the crystal form C has an X-ray powder diffraction pattern obtained using Cu Kα radiation with characteristic diffraction peaks at 20 angles of 7.18±0.20°, 8.47±0.20°, and 12.8±0.20°.

In some embodiments of the present disclosure, the above crystal form C has an X-ray powder diffraction pattern obtained using Cu Kα radiation with characteristic diffraction peaks at 20 angles of 7.18±0.20°, 8.47±0.20°, 12.80±0.20°, 15.80±0.20°, and 23.30±0.20°.

In some embodiments of the present disclosure, the above crystal form C has an X-ray powder diffraction pattern obtained using Cu Kα radiation with characteristic diffraction peaks at 20 angles of 7.18±0.20°, 8.47±0.20°, 11.55±0.20°, 12.80±0.20°, 15.80±0.20°, 16.96±0.20°, 19.23±0.20°, and 20.12±0.20°.

In some embodiments of the present disclosure, the above crystal form C has an X-ray powder diffraction pattern obtained using Cu Kα radiation with characteristic diffraction peaks at 20 angles of 7.18±0.20°, 8.47±0.20°, 11.55±0.20°, 12.80±0.20°, 15.80±0.20°, 16.96±0.20°, 19.23±0.20°, 20.12±0.20°, 23.30±0.20°, and 26.31±0.20°.

In some embodiments of the present disclosure, the above crystal form C has an X-ray powder diffraction pattern obtained using Cu Kα radiation with characteristic diffraction peaks at 20 angles of 7.18±0.20°, 8.47±0.20°, 12.80±0.20°, and/or 11.55±0.20°, and/or 15.80±0.20°, and/or 16.96±0.20°, and/or 19.23±0.20°, and/or 20.12±0.20°, and/or 23.30±0.20°, and/or 26.31±0.20°.

In some embodiments of the present disclosure, the above crystal form C has an X-ray powder diffraction pattern obtained using Cu Kα radiation with characteristic diffraction peaks at 20 angles of 7.18°, 8.47°, 11.55°, 12.80°, 15.80°, 16.96°, 19.23°, 20.12°, 23.30°, and 26.31°.

In some embodiments of the present disclosure, the XRPD pattern of the above crystal form C obtained using Cu Kα radiation is substantially as shown in Fig. 4.

In some embodiments of the present disclosure, in the XRPD pattern of the above crystal form C obtained using Cu Kα radiation, data of the diffraction peaks are as shown in Table 2:

**Table 2 XRPD data of the crystal form C of the compound of formula (I)**

| **No.** | 2θ[°] | Peak height [cts] | D-spacing [Å] | Relative intensity [%] |
|---|---|---|---|---|
| 1 | 7.1836 | 809.66 | 12.31 | 100.00 |
| 2 | 8.4748 | 302.29 | 10.43 | 37.34 |
| 3 | 11.5506 | 135.10 | 7.66 | 16.69 |
| 4 | 12.7958 | 463.54 | 6.92 | 57.25 |
| 5 | 15.7963 | 229.83 | 5.61 | 28.39 |
| 6 | 16.9615 | 156.58 | 5.23 | 19.34 |
| 7 | 19.2265 | 135.51 | 4.62 | 16.74 |
| 8 | 20.1160 | 170.60 | 4.41 | 21.07 |
| 9 | 23.2970 | 187.73 | 3.82 | 23.19 |
| 10 | 26.3104 | 109.49 | 3.39 | 13.52 |

In one embodiment of the present disclosure, the differential scanning calorimetry (DSC) curve of the above crystal form C shows a peak value of an exothermic peak at 225.1 °C±3 °C.

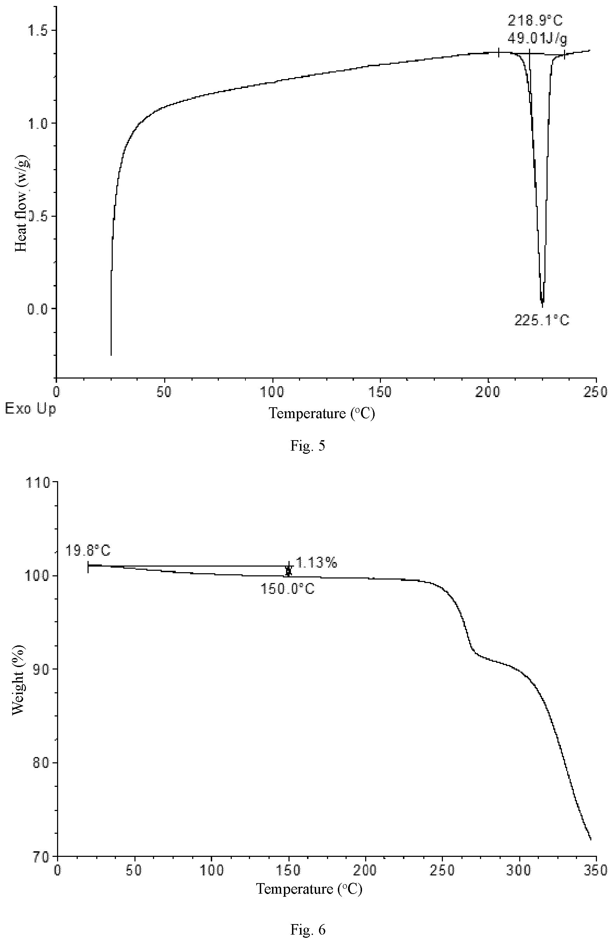

In one embodiment of the present disclosure, the DSC curve of the above crystal form C is substantially as shown in Fig. 5.

In one embodiment of the present disclosure, the thermogravimetric analysis (TGA) curve of the above crystal form C has a weight loss of 1.13% at 150 °C±3 °C.

In one embodiment of the present disclosure, the TGA curve of the above crystal form C is substantially as shown in Fig. 6.

The present disclosure also provides a crystal form A1 of the compound of formula (I), wherein the crystal form A1 has an X-ray powder diffraction pattern obtained using Cu Kα radiation with characteristic diffraction peaks at 20 angles of 7.15±0.20°, 9.13±0.20°, 11.01±0.20°, 21.60±0.20°, and 22.10±0.20°.

In some embodiments of the present disclosure, the above crystal form A1 has an X-ray powder diffraction pattern obtained using Cu Kα radiation with characteristic diffraction peaks at 20 angles of 7.15±0.20°, 9.13±0.20°, 11.01±0.20°, 16.15±0.20°, 21.60±0.20°, 22.10±0.20°, 23.66±0.20°, and 24.60±0.20°.

In some embodiments of the present disclosure, the above crystal form A1 has an X-ray powder diffraction pattern obtained using Cu Kα radiation with characteristic diffraction peaks at 20 angles of 7.15±0.20°, 9.13±0.20°, 9.71±0.20°, 11.01±0.20°, 11.81±0.20°, 15.17±0.20°, 16.15±0.20°, 21.60±0.20°, 22.10±0.20°, 23.01±0.20°, 23.66±0.20°, and 24.60±0.20°.

In some embodiments of the present disclosure, the above crystal form A1 has an X-ray powder diffraction pattern obtained using Cu Kα radiation with characteristic diffraction peaks at 20 angles of 7.15°, 7.85°, 9.13°, 9.71°, 11.01°, 11.81°, 12.63°, 13.12°, 15.17°, 15.72°, 16.15°, 16.90°, 17.43°, 18.30°, 18.81°, 19.32°, 19.67°, 20.39°, 21.60°, 22.10°, 23.01°, 23.66°, 24.60°, 25.40°, 26.17°, 26.94°, 27.42°, 28.16°, 29.94°, 30.54°, 32.96°, 33.50°, 35.54°, 37.22°, and 38.58°.

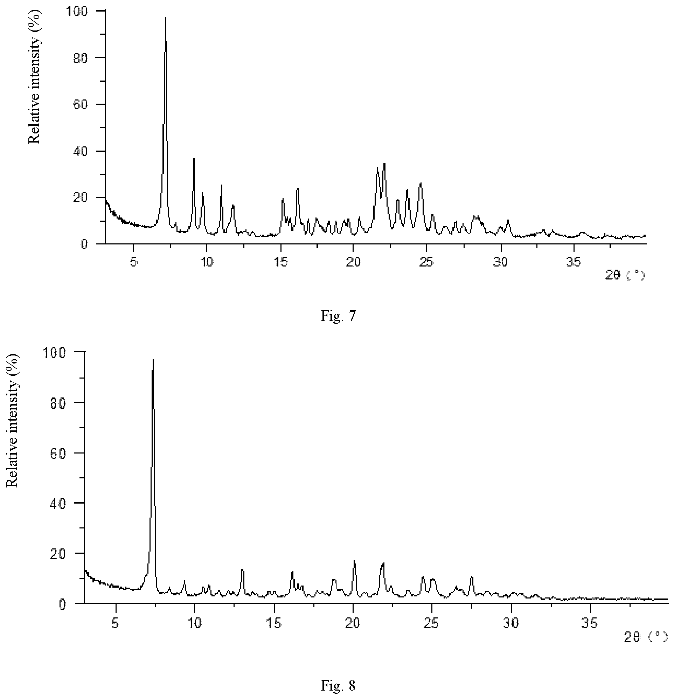

In some embodiments of the present disclosure, the XRPD pattern of the above crystal form A1 obtained using Cu Kα radiation is substantially as shown in Fig. 7.

In some embodiments of the present disclosure, in the XRPD pattern of the above crystal form A1 obtained using Cu Kα radiation, data of the diffraction peaks are as shown in Table 3:

**Table 3 XRPD data of the crystal form A1 of the compound of formula (I)**

| **No.** | 2θ[°] | Peak height [cts] | D-spacing [Å] | Relative intensity [%] |
|---|---|---|---|---|
| 1 | 7.1547 | 5243.4 | 12.36 | 100.00 |
| 2 | 7.8533 | 203.24 | 11.26 | 3.88 |
| 3 | 9.1274 | 1827.36 | 9.69 | 34.85 |
| 4 | 9.7122 | 948.46 | 9.11 | 18.09 |
| 5 | 11.0148 | 1270.53 | 8.03 | 24.23 |
| 6 | 11.8063 | 707.4 | 7.50 | 13.49 |
| 7 | 12.6328 | 139.85 | 7.01 | 2.67 |
| 8 | 13.1169 | 117.8 | 6.75 | 2.25 |
| 9 | 15.166 | 914.56 | 5.84 | 17.44 |
| 10 | 15.717 | 419.33 | 5.64 | 8.00 |
| 11 | 16.1478 | 1192.54 | 5.49 | 22.74 |
| 12 | 16.9048 | 391.32 | 5.24 | 7.46 |
| 13 | 17.4283 | 432.4 | 5.09 | 8.25 |
| 14 | 18.3039 | 341.81 | 4.85 | 6.52 |
| 15 | 18.8089 | 288.82 | 4.72 | 5.51 |
| 16 | 19.319 | 327.77 | 4.59 | 6.25 |
| 17 | 19.6703 | 338.78 | 4.51 | 6.46 |
| 18 | 20.3918 | 419.96 | 4.36 | 8.01 |
| 19 | 21.5978 | 1663.42 | 4.11 | 31.72 |
| 20 | 22.0973 | 1772.04 | 4.02 | 33.80 |
| 21 | 23.0083 | 849.73 | 3.87 | 16.21 |
| 22 | 23.6583 | 1101.06 | 3.76 | 21.00 |
| 23 | 24.5952 | 1239.79 | 3.62 | 23.64 |
| 24 | 25.3972 | 480.55 | 3.51 | 9.16 |
| 25 | 26.1724 | 184.87 | 3.40 | 3.53 |
| 26 | 26.938 | 342.05 | 3.31 | 6.52 |
| 27 | 27.416 | 257.91 | 3.25 | 4.92 |
| 28 | 28.16 | 451.58 | 3.17 | 8.61 |
| 29 | 29.9376 | 222.75 | 2.98 | 4.25 |
| 30 | 30.536 | 376.26 | 2.93 | 7.18 |
| 31 | 32.9615 | 193.49 | 2.72 | 3.69 |
| 32 | 33.4971 | 163.51 | 2.68 | 3.12 |
| 33 | 35.5373 | 146.83 | 2.53 | 2.80 |
| 34 | 37.2248 | 74.92 | 2.42 | 1.43 |
| 35 | 38.5826 | 49.07 | 2.33 | 0.94 |

The present disclosure also provides a crystal form D of the compound of formula (I), wherein the crystal form D has an X-ray powder diffraction pattern obtained using Cu Kα radiation with characteristic diffraction peaks at 20 angles of 7.35±0.20°, 13.02±0.20°, 16.18±0.20°, 20.10±0.20°, and 21.91±0.20°.

In some embodiments of the present disclosure, the above crystal form D has an X-ray powder diffraction pattern obtained using Cu Kα radiation with characteristic diffraction peaks at 20 angles of 7.35±0.20°, 13.02±0.20°, 16.18±0.20°, 20.10±0.20°, 21.91±0.20°, 24.41±0.20°, 25.03±0.20°, and 27.49±0.20°.

In some embodiments of the present disclosure, the above crystal form D has an X-ray powder diffraction pattern obtained using Cu Kα radiation with characteristic diffraction peaks at 20 angles of 7.35±0.20°, 9.36±0.20°, 10.90±0.20°, 13.02±0.20°, 16.18±0.20°, 18.80±0.20°, 20.10±0.20°, 21.91±0.20°, 22.40±0.20°, 24.41±0.20°, 25.03±0.20°, and 27.49±0.20°.

In some embodiments of the present disclosure, the above crystal form D has an X-ray powder diffraction pattern obtained using Cu Kα radiation with characteristic diffraction peaks at 20 angles of 7.35°, 8.41°, 9.36°, 10.49°, 10.90°, 11.55°, 12.11°, 13.02°, 13.79°, 14.68°, 15.07°, 16.18°, 16.53°, 16.77°, 17.83°, 18.80°, 20.10°, 20.75°, 21.75°, 21.91°, 22.40°, 23.45°, 24.41°, 25.03°, 26.48°, 26.86°, 27.49°, 28.45°, and 29.04°.

In some embodiments of the present disclosure, the above crystal form D has an X-ray powder diffraction pattern obtained using Cu Kα radiation with characteristic diffraction peaks at 20 angles of 7.35°, 8.41°, 9.36°, 10.49°, 10.90°, 11.55°, 12.11°, 13.02°, 13.79°, 14.68°, 15.07°, 16.18°, 16.53°, 16.77°, 17.83°, 18.80°, 20.10°, 20.75°, 21.75°, 21.91°, 22.40°, 23.45°, 24.41°, 25.03°, 26.48°, 26.86°, 27.49°, 28.45°, 29.04°, 30.16°, 30.67°, 31.56°, and 34.26°.

In some embodiments of the present disclosure, the XRPD pattern of the above crystal form D obtained using Cu Kα radiation is substantially as shown in Fig. 8.

In some embodiments of the present disclosure, in the XRPD pattern of the above crystal form D obtained using Cu Kα radiation, data of the diffraction peaks are as shown in Table 4:

**Table 4 XRPD data of the crystal form D of the compound of formula (I)**

| **No.** | 2θ[°] | Peak height [cts] | D-spacing [Å] | Relative intensity [%] |
|---|---|---|---|---|
| 1 | 7.3536 | 7424.20 | 12.02 | 100.00 |
| 2 | 8.4059 | 193.22 | 10.52 | 2.60 |
| 3 | 9.3633 | 444.05 | 9.45 | 5.98 |
| 4 | 10.4885 | 297.47 | 8.43 | 4.01 |
| 5 | 10.8998 | 382.39 | 8.12 | 5.15 |
| 6 | 11.5534 | 224.38 | 7.66 | 3.02 |
| 7 | 12.1052 | 196.50 | 7.31 | 2.65 |
| 8 | 13.0168 | 895.98 | 6.80 | 12.07 |
| 9 | 13.7880 | 99.41 | 6.42 | 1.34 |
| 10 | 14.6811 | 192.67 | 6.03 | 2.60 |
| 11 | 15.0709 | 179.33 | 5.88 | 2.42 |
| 12 | 16.1836 | 824.30 | 5.48 | 11.10 |
| 13 | 16.5306 | 419.38 | 5.36 | 5.65 |
| 14 | 16.7680 | 374.78 | 5.29 | 5.05 |
| 15 | 17.8279 | 151.96 | 4.98 | 2.05 |
| 16 | 18.7964 | 576.47 | 4.72 | 7.76 |
| 17 | 20.1003 | 1164.60 | 4.42 | 15.69 |
| 18 | 20.7532 | 126.41 | 4.28 | 1.70 |
| 19 | 21.7468 | 888.00 | 4.09 | 11.96 |
| 20 | 21.9120 | 1114.17 | 4.06 | 15.01 |
| 21 | 22.4001 | 375.62 | 3.97 | 5.06 |
| 22 | 23.4468 | 218.72 | 3.79 | 2.95 |
| 23 | 24.4050 | 659.41 | 3.65 | 8.88 |
| 24 | 25.0309 | 583.13 | 3.56 | 7.85 |
| 25 | 26.4846 | 360.94 | 3.37 | 4.86 |
| 26 | 26.8557 | 265.41 | 3.32 | 3.57 |
| 27 | 27.4917 | 694.29 | 3.24 | 9.35 |
| 28 | 28.4548 | 221.36 | 3.14 | 2.98 |
| 29 | 29.0399 | 163.18 | 3.07 | 2.20 |
| 30 | 30.1629 | 207.14 | 2.96 | 2.79 |
| 31 | 30.6696 | 154.08 | 2.92 | 2.08 |
| 32 | 31.5640 | 154.79 | 2.83 | 2.08 |
| 33 | 34.2623 | 57.49 | 2.62 | 0.77 |

In one embodiment of the present disclosure, the differential scanning calorimetry (DSC) curve of the above crystal form D shows a peak value of an exothermic peak at 220.0 °C±3 °C.

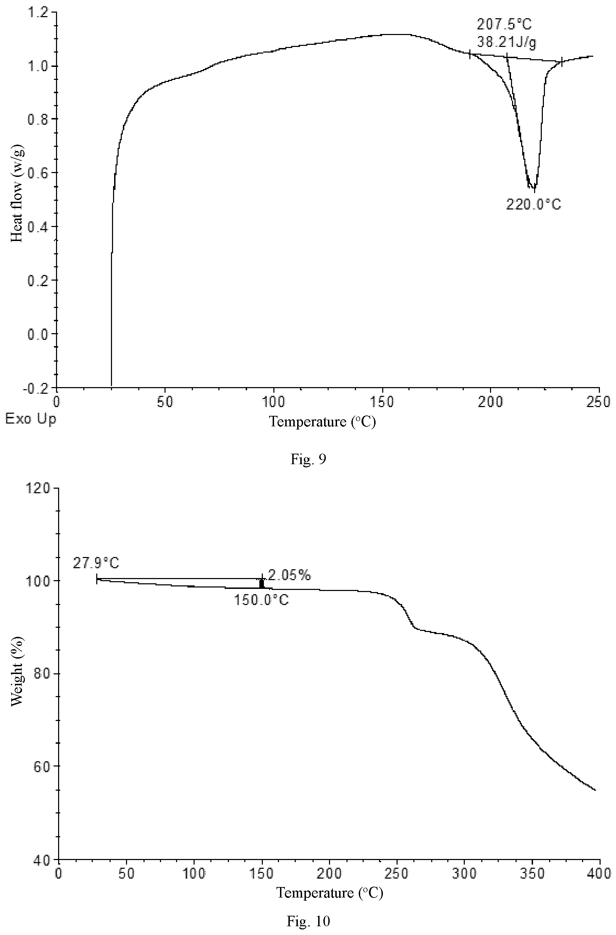

In one embodiment of the present disclosure, the DSC curve of the above crystal form D is substantially as shown in Fig. 9.

In one embodiment of the present disclosure, the thermogravimetric analysis (TGA) curve of the above crystal form D has a weight loss of 2.05% at 150.0 °C±3 °C.

In one embodiment of the present disclosure, the TGA curve of the above crystal form D is substantially as shown in Fig. 10.

The present disclosure also provides a crystal form E of the compound of formula (I), wherein the crystal form E has an X-ray powder diffraction pattern obtained using Cu Kα radiation with characteristic diffraction peaks at 20 angles of 4.49±0.20°, 6.15±0.20°, 13.40±0.20°, 17.26±0.20°, and 26.04±0.20°.

In some embodiments of the present disclosure, the above crystal form E has an X-ray powder diffraction pattern obtained using Cu Kα radiation with characteristic diffraction peaks at 20 angles of 4.49±0.20°, 6.15±0.20°, 8.92±0.20°, 11.47±0.20°, 12.30±0.20°, 13.40±0.20°, 17.26±0.20°, and 26.04±0.20°.

In some embodiments of the present disclosure, the above crystal form E has an X-ray powder diffraction pattern obtained using Cu Kα radiation with characteristic diffraction peaks at 20 angles of 4.49±0.20°, 6.15±0.20°, 8.92±0.20°, 11.47±0.20°, 12.30±0.20°, 13.40±0.20°, 16.56±0.20°, 17.26±0.20°, 19.03±0.20°, 21.01±0.20°, 26.04±0.20°, and 26.60±0.20°.

In some embodiments of the present disclosure, the above crystal form E has an X-ray powder diffraction pattern obtained using Cu Kα radiation with characteristic diffraction peaks at 20 angles of 4.49°, 6.15°, 8.92°, 11.47°, 12.30°, 13.40°, 14.59°, 16.56°, 17.26°, 19.03°, 21.01°, 22.16°, 23.88°, 24.54°, 26.04°, 26.60°, 27.47°, and 27.96°.

In some embodiments of the present disclosure, the above crystal form E has an X-ray powder diffraction pattern obtained using Cu Kα radiation with characteristic diffraction peaks at 20 angles of 4.49°, 6.15°, 8.92°, 11.47°, 12.30°, 13.40°, 14.59°, 16.56°, 17.26°, 19.03°, 21.01°, 22.16°, 23.88°, 24.54°, 26.04°, 26.60°, 27.47°, 27.96°, 30.60°, 31.71°, 33.61°, and 37.65°.

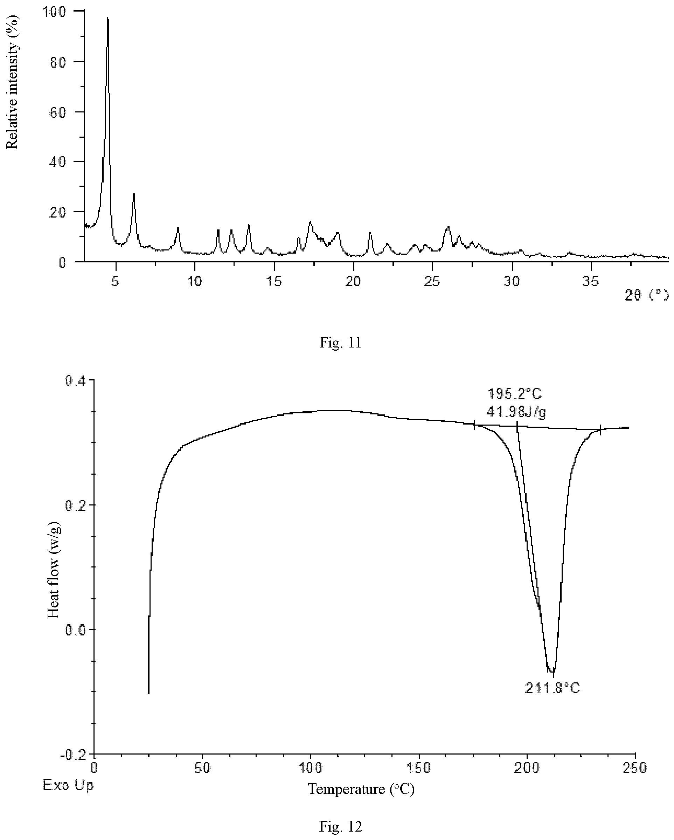

In some embodiments of the present disclosure, the XRPD pattern of the above crystal form E obtained using Cu Kα radiation is substantially as shown in Fig. 11.

In some embodiments of the present disclosure, in the XRPD pattern of the above crystal form E obtained using Cu Kα radiation, data of the diffraction peaks are as shown in Table 5:

**Table 5 XRPD data of the crystal form E of the compound of formula (I)**

| **No.** | 2θ[°] | Peak height [cts] | D-spacing [Å] | Relative intensity [%] |
|---|---|---|---|---|
| 1 | 4.4892 | 6698.37 | 19.68 | 100.00 |
| 2 | 6.1517 | 1627.46 | 14.37 | 24.30 |
| 3 | 8.9240 | 741.97 | 9.91 | 11.08 |
| 4 | 11.4651 | 747.07 | 7.72 | 11.15 |
| 5 | 12.3004 | 781.86 | 7.20 | 11.67 |
| 6 | 13.3971 | 923.52 | 6.61 | 13.79 |
| 7 | 14.5899 | 211.88 | 6.07 | 3.16 |
| 8 | 16.5557 | 518.85 | 5.35 | 7.75 |
| 9 | 17.2588 | 1013.47 | 5.14 | 15.13 |
| 10 | 19.0287 | 721.66 | 4.66 | 10.77 |
| 11 | 21.0068 | 738.37 | 4.23 | 11.02 |
| 12 | 22.1602 | 362.74 | 4.01 | 5.42 |
| 13 | 23.8815 | 306.05 | 3.73 | 4.57 |
| 14 | 24.5384 | 312.65 | 3.63 | 4.67 |
| 15 | 26.0409 | 791.75 | 3.42 | 11.82 |
| 16 | 26.6003 | 562.16 | 3.35 | 8.39 |
| 17 | 27.4661 | 397.58 | 3.25 | 5.94 |
| 18 | 27.9643 | 336.90 | 3.19 | 5.03 |
| 19 | 30.5974 | 185.03 | 2.92 | 2.76 |
| 20 | 31.7122 | 95.74 | 2.82 | 1.43 |
| 21 | 33.6097 | 137.54 | 2.67 | 2.05 |
| 22 | 37.6458 | 91.61 | 2.39 | 1.37 |

In one embodiment of the present disclosure, the differential scanning calorimetry (DSC) curve of the above crystal form E shows a peak value of an exothermic peak at 211.8 °C±3 °C.

In one embodiment of the present disclosure, the DSC curve of the above crystal form E is substantially as shown in Fig. 12.

In one embodiment of the present disclosure, the thermogravimetric analysis (TGA) curve of the above crystal form E has a weight loss of 2.61% at 150.0 °C±3 °C.

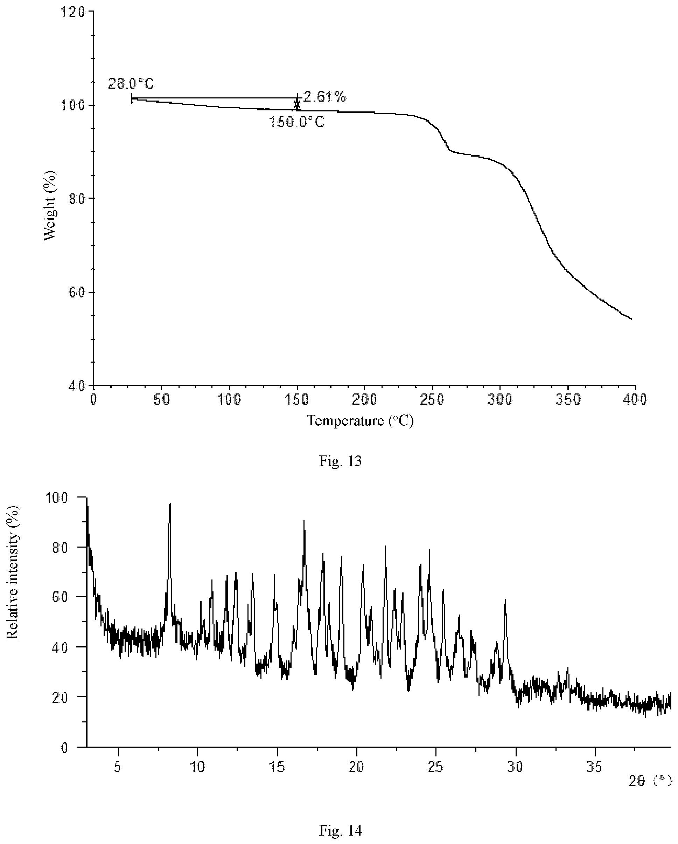

In one embodiment of the present disclosure, the TGA curve of the above crystal form E is substantially as shown in Fig. 13.

The present disclosure also provides a crystal form F of the compound of formula (I), wherein the crystal form F has an X-ray powder diffraction pattern obtained using Cu Kα radiation with characteristic diffraction peaks at 20 angles of 8.20±0.20°, 16.73±0.20°, 21.84±0.20°, 23.99±0.20°, and 24.56±0.20°.

In some embodiments of the present disclosure, the above crystal form F has an X-ray powder diffraction pattern obtained using Cu Kα radiation with characteristic diffraction peaks at 20 angles of 8.20±0.20°, 16.73±0.20°, 17.90±0.20°, 19.03±0.20°, 20.38±0.20°, 21.84±0.20°, 23.99±0.20°, and 24.56±0.20°.

In some embodiments of the present disclosure, the above crystal form F has an X-ray powder diffraction pattern obtained using Cu Kα radiation with characteristic diffraction peaks at 20 angles of 8.20±0.20°, 13.49±0.20°, 16.73±0.20°, 17.90±0.20°, 19.03±0.20°, 20.38±0.20°, 21.84±0.20°, 22.38±0.20°, 23.99±0.20°, 24.56±0.20°, 25.45±0.20°, and 29.34±0.20°.

In some embodiments of the present disclosure, the above crystal form F has an X-ray powder diffraction pattern obtained using Cu Kα radiation with characteristic diffraction peaks at 2θ angles of 8.20°, 10.86°, 11.79°, 12.41°, 13.49°, 14.79°, 16.73°, 17.90°, 19.03°, 20.38°, 21.84°, 22.38°, 22.91°, 23.99°, 24.56°, 25.45°, 26.43°, 27.33°, 28.76°, and 29.34°.

In some embodiments of the present disclosure, the XRPD pattern of the above crystal form F obtained using Cu Kα radiation is substantially as shown in Fig. 14.

In some embodiments of the present disclosure, in the XRPD pattern of the above crystal form F obtained using Cu Kα radiation, data of the diffraction peaks are as shown in Table 6:

**Table 6 XRPD data of the crystal form F of the compound of formula (I)**

| **No.** | 2θ[°] | Peak height [cts] | D-spacing [Å] | Relative intensity [%] |
|---|---|---|---|---|
| 1 | 8.1989 | 297.16 | 10.78 | 100.00 |
| 2 | 10.8648 | 142.43 | 8.14 | 47.93 |
| 3 | 11.7912 | 143.16 | 7.51 | 48.18 |
| 4 | 12.4117 | 168.01 | 7.13 | 56.54 |
| 5 | 13.4865 | 169.65 | 6.57 | 57.09 |
| 6 | 14.7927 | 154.54 | 5.99 | 52.01 |
| 7 | 16.7345 | 251.21 | 5.30 | 84.54 |
| 8 | 17.9025 | 216.97 | 4.95 | 73.01 |
| 9 | 19.0262 | 227.77 | 4.66 | 76.65 |
| 10 | 20.3761 | 210.54 | 4.36 | 70.85 |
| 11 | 21.8395 | 243.47 | 4.07 | 81.93 |
| 12 | 22.3811 | 176.11 | 3.97 | 59.26 |
| 13 | 22.9104 | 162.97 | 3.88 | 54.84 |
| 14 | 23.9905 | 238.48 | 3.71 | 80.25 |
| 15 | 24.5598 | 234.91 | 3.62 | 79.05 |
| 16 | 25.4508 | 186.56 | 3.50 | 62.78 |
| 17 | 26.4299 | 113.07 | 3.37 | 38.05 |
| 18 | 27.3339 | 91.24 | 3.26 | 30.71 |
| 19 | 28.7636 | 93.11 | 3.10 | 31.33 |
| 20 | 29.3388 | 180.07 | 3.04 | 60.60 |

In one embodiment of the present disclosure, the differential scanning calorimetry (DSC) curve of the above crystal form F shows a peak value of an exothermic peak at 225.8 °C±3 °C.

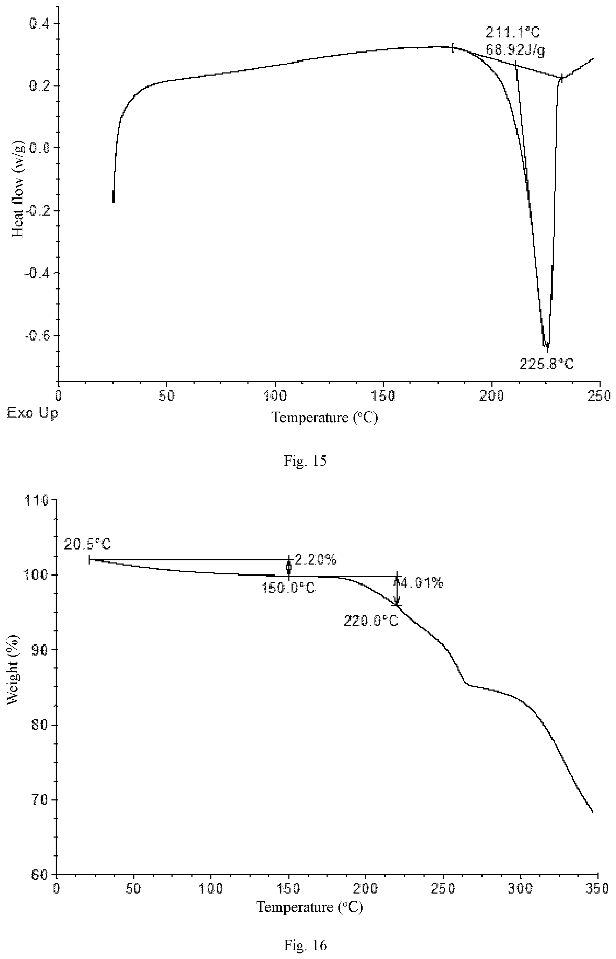

In one embodiment of the present disclosure, the DSC curve of the above crystal form F is substantially as shown in Fig. 15.

In one embodiment of the present disclosure, the thermogravimetric analysis (TGA) curve of the above crystal form F has a weight loss of 2.20% at 150.0 °C±3 °C.

In one embodiment of the present disclosure, the thermogravimetric analysis (TGA) curve of the above crystal form F has a weight loss of 2.20% at 150.0 °C±3 °C, and continues to have a weight loss of 4.01% at 220.0 °C±3 °C.

In one embodiment of the present disclosure, the TGA curve of the above crystal form F is substantially as shown in Fig. 16.

The present disclosure also provides a crystal form G of the compound of formula (I), wherein the crystal form G has an X-ray powder diffraction pattern obtained using Cu Kα radiation with characteristic diffraction peaks at 20 angles of 8.55±0.20°, 12.62±0.20°, 24.96±0.20°, and 25.39±0.20°.

In some embodiments of the present disclosure, the above crystal form G has an X-ray powder diffraction pattern obtained using Cu Kα radiation with characteristic diffraction peaks at 20 angles of 8.55±0.20°, 12.62±0.20°, 14.95±0.20°, 15.64±0.20°, 19.90±0.20°, 24.96±0.20°, 25.39±0.20°, and 26.90±0.20°.

In some embodiments of the present disclosure, the above crystal form G has an X-ray powder diffraction pattern obtained using Cu Kα radiation with characteristic diffraction peaks at 20 angles of 8.55±0.20°, 12.62±0.20°, 14.95±0.20°, 15.64±0.20°, 18.87±0.20°, 19.90±0.20°, 22.54±0.20°, 24.96±0.20°, 25.39±0.20°, 25.85±0.20°, 26.90±0.20°, and 28.15±0.20°.

In some embodiments of the present disclosure, the above crystal form G has an X-ray powder diffraction pattern obtained using Cu Kα radiation with characteristic diffraction peaks at 20 angles of 6.34°, 8.31°, 8.55°, 9.05°, 10.71°, 11.67°, 12.22°, 12.62°, 12.86°, 14.18°, 14.95°, 15.64°, 16.02°, 16.49°, 17.15°, 17.66°, 18.16°, 18.87°, 19.34°, 19.90°, 21.16°, 21.49°, 22.54°, 22.77°, 23.34°, 24.20°, 24.61°, 24.96°, 25.39°, 25.85°, 26.90°, 28.15°, 29.12°, and 29.80°.

In some embodiments of the present disclosure, the above crystal form G has an X-ray powder diffraction pattern obtained using Cu Kα radiation with characteristic diffraction peaks at 20 angles of 6.34°, 8.31°, 8.55°, 9.05°, 10.71°, 11.67°, 12.22°, 12.62°, 12.86°, 14.18°, 14.95°, 15.64°, 16.02°, 16.49°, 17.15°, 17.66°, 18.16°, 18.87°, 19.34°, 19.90°, 21.16°, 21.49°, 22.54°, 22.77°, 23.34°, 24.20°, 24.61°, 24.96°, 25.39°, 25.85°, 26.90°, 28.15°, 29.12°, 29.80°, 30.59°, 31.76°, 32.57 °, 33.72°, 35.90°, and 37.86°.

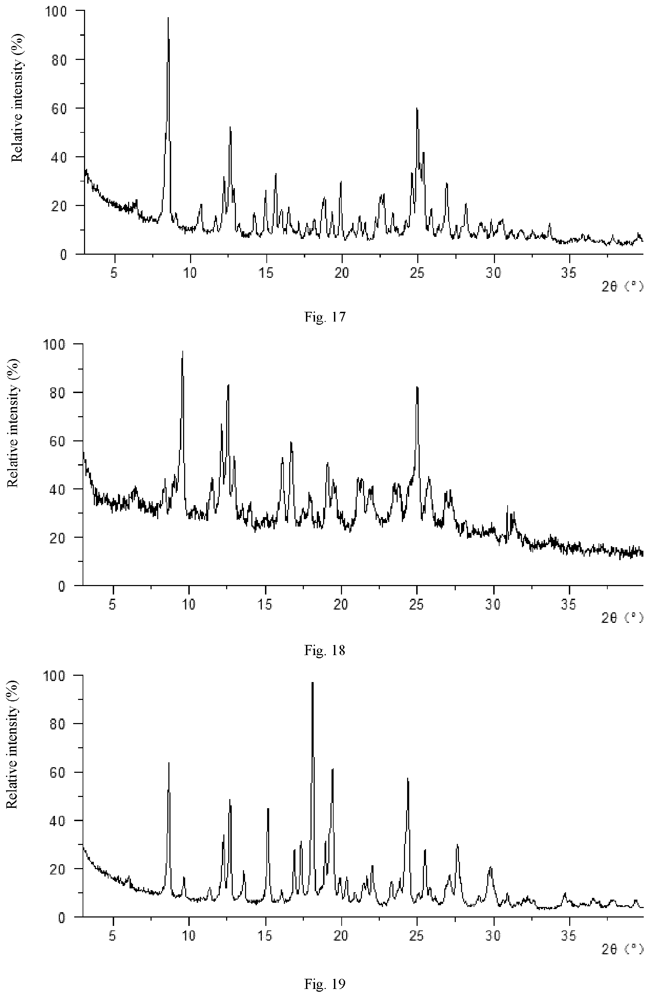

In some embodiments of the present disclosure, the XRPD pattern of the above crystal form G obtained using Cu Kα radiation is substantially as shown in Fig. 17.

In some embodiments of the present disclosure, in the XRPD pattern of the above crystal form G obtained using Cu Kα radiation, data of the diffraction peaks are as shown in Table 7:

**Table 7 XRPD data of the crystal form G of the compound of formula (I)**

| **No.** | 2θ[°] | Peak height [cts] | D-spacing [Å] | Relative intensity [%] |
|---|---|---|---|---|
| 1 | 6.3390 | 131.38 | 13.94 | 5.95 |
| 2 | 8.3100 | 928.32 | 10.64 | 42.07 |
| 3 | 8.5500 | 2206.41 | 10.34 | 100.00 |
| 4 | 9.0489 | 130.69 | 9.77 | 5.92 |
| 5 | 10.7067 | 297.30 | 8.26 | 13.47 |
| 6 | 11.6672 | 174.91 | 7.58 | 7.93 |
| 7 | 12.2189 | 586.63 | 7.24 | 26.59 |
| 8 | 12.6223 | 1123.66 | 7.01 | 50.93 |
| 9 | 12.8564 | 481.07 | 6.89 | 21.80 |
| 10 | 14.1770 | 204.15 | 6.25 | 9.25 |
| 11 | 14.9500 | 473.04 | 5.93 | 21.44 |
| 12 | 15.6434 | 653.89 | 5.66 | 29.64 |
| 13 | 16.0173 | 272.10 | 5.53 | 12.33 |
| 14 | 16.4906 | 300.48 | 5.38 | 13.62 |
| 15 | 17.1469 | 134.52 | 5.17 | 6.10 |
| 16 | 17.6551 | 119.86 | 5.02 | 5.43 |
| 17 | 18.1608 | 180.76 | 4.88 | 8.19 |
| 18 | 18.8675 | 427.83 | 4.70 | 19.39 |
| 19 | 19.3424 | 266.55 | 4.59 | 12.08 |
| 20 | 19.8999 | 576.83 | 4.46 | 26.14 |
| 21 | 21.1631 | 247.74 | 4.20 | 11.23 |
| 22 | 21.4937 | 178.99 | 4.13 | 8.11 |
| 23 | 22.5365 | 458.09 | 3.95 | 20.76 |
| 24 | 22.7681 | 456.95 | 3.91 | 20.71 |
| 25 | 23.3426 | 266.43 | 3.81 | 12.08 |
| 26 | 24.1992 | 161.47 | 3.68 | 7.32 |
| 27 | 24.6147 | 682.13 | 3.62 | 30.92 |
| 28 | 24.9597 | 1375.40 | 3.57 | 62.34 |
| 29 | 25.3940 | 801.94 | 3.51 | 36.35 |
| 30 | 25.8537 | 301.56 | 3.45 | 13.67 |
| 31 | 26.8983 | 552.00 | 3.31 | 25.02 |
| 32 | 28.1500 | 372.89 | 3.17 | 16.90 |
| 33 | 29.1169 | 152.70 | 3.07 | 6.92 |
| 34 | 29.8037 | 177.11 | 3.00 | 8.03 |
| 35 | 30.5929 | 197.20 | 2.92 | 8.94 |
| 36 | 31.7605 | 113.70 | 2.82 | 5.15 |
| 37 | 32.5730 | 98.52 | 2.75 | 4.47 |
| 38 | 33.7173 | 187.70 | 2.66 | 8.51 |
| 39 | 35.8968 | 57.54 | 2.50 | 2.61 |
| 40 | 37.8595 | 85.83 | 2.38 | 3.89 |

The present disclosure also provides a crystal form H of the compound of formula (I), wherein the crystal form H has an X-ray powder diffraction pattern obtained using Cu Kα radiation with characteristic diffraction peaks at 20 angles of 9.57±0.20°, 12.16±0.20°, 12.58±0.20°, 16.74±0.20°, and 25.02±0.20°.

In some embodiments of the present disclosure, the above crystal form H has an X-ray powder diffraction pattern obtained using Cu Kα radiation with characteristic diffraction peaks at 2θ angles of 9.57±0.20°, 12.16±0.20°, 12.58±0.20°, 16.14±0.20°, 16.74±0.20°, 19.10±0.20°, 25.02±0.20°, and 25.79±0.20°.

In some embodiments of the present disclosure, the above crystal form H has an X-ray powder diffraction pattern obtained using Cu Kα radiation with characteristic diffraction peaks at 2θ angles of 9.57±0.20°, 11.52±0.20°, 12.16±0.20°, 12.58±0.20°, 16.14±0.20°, 16.74±0.20°, 19.10±0.20°, 21.39±0.20°, 23.69±0.20°, 25.02±0.20°, 25.79±0.20°, and 26.86±0.20°.

In some embodiments of the present disclosure, the above crystal form H has an X-ray powder diffraction pattern obtained using Cu Kα radiation with characteristic diffraction peaks at 20 angles of 6.42°, 8.37°, 9.57°, 11.52°, 12.16°, 12.58°, 12.97°, 13.99°, 16.14°, 16.74°, 17.94°, 19.10°, 19.46°, 21.07°, 21.39°, 21.96°, 23.69°, 25.02°, 25.79°, and 26.86°.

In some embodiments of the present disclosure, the XRPD pattern of the above crystal form H obtained using Cu Kα radiation is substantially as shown in Fig. 18.

In some embodiments of the present disclosure, in the XRPD pattern of the above crystal form H obtained using Cu Kα radiation, data of the diffraction peaks are as shown in Table 8:

**Table 8 XRPD data of the crystal form H of the compound of formula (I)**

| **No.** | 2θ[°] | Peak height [cts] | D-spacing [Å] | Relative intensity [%] |
|---|---|---|---|---|
| 1 | 6.4202 | 101.81 | 13.77 | 10.84 |
| 2 | 8.3698 | 172.44 | 10.56 | 18.36 |
| 3 | 9.5654 | 939.14 | 9.25 | 100.00 |
| 4 | 11.5243 | 249.78 | 7.68 | 26.60 |
| 5 | 12.1638 | 536.55 | 7.28 | 57.13 |
| 6 | 12.5758 | 785.85 | 7.04 | 83.68 |
| 7 | 12.9685 | 387.92 | 6.83 | 41.31 |
| 8 | 13.9912 | 126.57 | 6.33 | 13.48 |
| 9 | 16.1427 | 371.82 | 5.49 | 39.59 |
| 10 | 16.7443 | 485.10 | 5.29 | 51.65 |
| 11 | 17.9412 | 174.74 | 4.94 | 18.61 |
| 12 | 19.1006 | 368.73 | 4.65 | 39.26 |
| 13 | 19.4609 | 261.19 | 4.56 | 27.81 |
| 14 | 21.0739 | 245.39 | 4.22 | 26.13 |
| 15 | 21.3889 | 259.47 | 4.15 | 27.63 |
| 16 | 21.9595 | 201.77 | 4.05 | 21.48 |
| 17 | 23.6908 | 214.83 | 3.76 | 22.88 |
| 18 | 25.0239 | 820.99 | 3.56 | 87.42 |
| 19 | 25.7893 | 299.00 | 3.45 | 31.84 |
| 20 | 26.8576 | 210.32 | 3.32 | 22.40 |
| 21 | 30.9168 | 183.43 | 2.89 | 19.53 |
| 22 | 31.3679 | 136.03 | 2.85 | 14.48 |

The present disclosure also provides a pharmaceutically acceptable salt of the compound of formula (I), 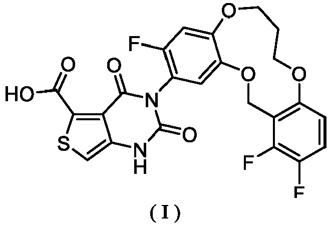 wherein the pharmaceutically acceptable salt of the compound is a lysine salt, a dibenzylethylenediamine salt, a choline salt, a meglumine salt, a triethylamine salt, an aluminum salt, a zinc salt or a lithium salt.

In some embodiments of the present disclosure, the structure of the above choline salt of the compound of formula (I) is shown in formula (II), and the structure of the above dibenzylethylenediamine salt of the compound of formula (I) is shown in formula (IV), 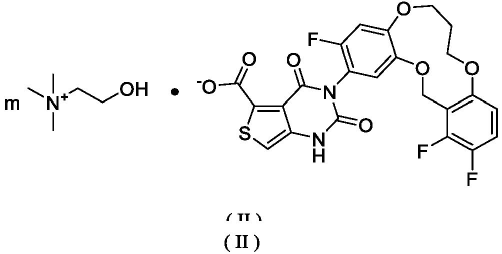 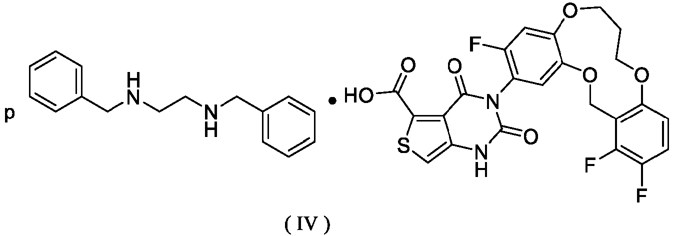 wherein, m is selected from 0.5 to 1.5 and p is selected from 0.4 to 1.5. In some embodiments of the present disclosure, the above m is selected from 0.5, 0.6, 0.7, 0.8, 0.9, 1.0, 1.1, 1.2, 1.3, 1.4, and 1.5.

In some embodiments of the present disclosure, the above m is selected from 0.8, 0.9, 1.0, 1.1, and 1.2.

In some embodiments of the present disclosure, the above m is 1.0.

In some embodiments of the present disclosure, the above p is selected from 0.4, 0.5, 0.6, 0.7, 0.8, 0.9, 1.0, 1.1, 1.2, 1.3, 1.4, and 1.5.

In some embodiments of the present disclosure, the above p is selected from 0.4, 0.5, 0.6, 0.9, 1.0, and 1.1.

In some embodiments of the present disclosure, the above p is selected from 0.5 and 1.0.

In some embodiments of the present disclosure, the above compound of formula (II) is a compound of formula (II-1),

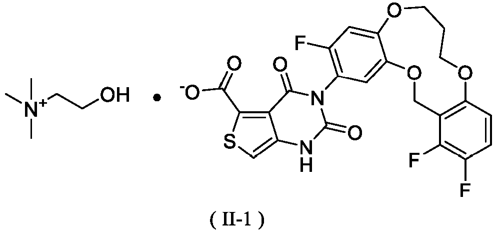

The present disclosure also provides a crystal form S1 of the compound of formula (II-1), wherein the crystal form S1 has an X-ray powder diffraction pattern obtained using Cu Kα radiation with characteristic diffraction peaks at 20 angles of 8.68±0.20°, 12.70±0.20°, 18.12±0.20°, 19.43±0.20°, and 24.41±0.20°.

In some embodiments of the present disclosure, the above crystal form S1 has an X-ray powder diffraction pattern obtained using Cu Kα radiation with characteristic diffraction peaks at 20 angles of 8.68±0.20°, 12.27±0.20°, 12.70±0.20°, 15.20±0.20°, 18.12±0.20°, 18.95±0.20°, 19.43±0.20°, and 24.41±0.20°.

In some embodiments of the present disclosure, the above crystal form S1 has an X-ray powder diffraction pattern obtained using Cu Kα radiation with characteristic diffraction peaks at 20 angles of 8.68±0.20°, 12.27±0.20°, 12.70±0.20°, 15.20±0.20°, 16.94±0.20°, 17.37±0.20°, 18.12±0.20°, 18.95±0.20°, 19.43±0.20°, 24.41±0.20°, 25.51±0.20°, and 27.62±0.20°.

In some embodiments of the present disclosure, the above crystal form S1 has an X-ray powder diffraction pattern obtained using Cu Kα radiation with characteristic diffraction peaks at 20 angles of 5.98°, 8.68°, 9.67°, 11.39°, 12.27°, 12.70°, 13.61°, 15.20°, 16.09°, 16.94°, 17.37°, 18.12°, 18.95°, 19.43°, 19.93°, 20.35°, 20.91°, 21.44°, 21.69°, 22.05°, 23.32°, 23.85°, 24.41°, 25.51°, 27.11°, 27.62°, 29.05°, and 29.85°.

In some embodiments of the present disclosure, the above crystal form S1 has an X-ray powder diffraction pattern obtained using Cu Kα radiation with characteristic diffraction peaks at 20 angles of 5.98°, 8.68°, 9.67°, 11.39°, 12.27°, 12.70°, 13.61°, 15.20°, 16.09°, 16.94°, 17.37°, 18.12°, 18.95°, 19.43°, 19.93°, 20.35°, 20.91°, 21.44°, 21.69°, 22.05°, 23.32°, 23.85°, 24.41°, 25.51°, 27.11°, 27.62°, 29.05°, 29.85°, 30.91°, 32.34°, 32.68°, 34.67°, 36.58°, 37.86°, and 39.38°.

In some embodiments of the present disclosure, the XRPD pattern of the above crystal form S1 obtained using Cu Kα radiation is substantially as shown in Fig. 19.

In some embodiments of the present disclosure, in the XRPD pattern of the above crystal form S1 obtained using Cu Kα radiation, data of the diffraction peaks are as shown in Table 9:

**Table 9 XRPD data of the crystal form S1 of the compound of formula (II-1)**

| **No.** | 2θ[°] | Peak height [cts] | D-spacing [Å] | Relative intensity [%] |
|---|---|---|---|---|
| 1 | 5.9826 | 104.23 | 14.77 | 3.45 |
| 2 | 8.6824 | 1869.35 | 10.18 | 61.94 |
| 3 | 9.6684 | 291.64 | 9.15 | 9.66 |
| 4 | 11.3851 | 173.31 | 7.77 | 5.74 |
| 5 | 12.2664 | 930.12 | 7.22 | 30.82 |
| 6 | 12.6968 | 1415.99 | 6.97 | 46.92 |
| 7 | 13.6102 | 405.65 | 6.51 | 13.44 |
| 8 | 15.2027 | 1306.30 | 5.83 | 43.28 |
| 9 | 16.0867 | 155.98 | 5.51 | 5.17 |
| 10 | 16.9416 | 737.19 | 5.23 | 24.43 |
| 11 | 17.3740 | 835.52 | 5.10 | 27.68 |
| 12 | 18.1201 | 3018.11 | 4.90 | 100.00 |
| 13 | 18.9517 | 866.34 | 4.68 | 28.70 |
| 14 | 19.4287 | 1850.65 | 4.57 | 61.32 |
| 15 | 19.9261 | 330.66 | 4.46 | 10.96 |
| 16 | 20.3473 | 362.08 | 4.36 | 12.00 |
| 17 | 20.9073 | 156.84 | 4.25 | 5.20 |
| 18 | 21.4435 | 274.07 | 4.14 | 9.08 |
| 19 | 21.6905 | 388.25 | 4.10 | 12.86 |
| 20 | 22.0454 | 544.01 | 4.03 | 18.02 |
| 21 | 23.3178 | 323.86 | 3.81 | 10.73 |
| 22 | 23.8493 | 363.18 | 3.73 | 12.03 |
| 23 | 24.4113 | 1686.89 | 3.65 | 55.89 |
| 24 | 25.5099 | 776.37 | 3.49 | 25.72 |
| 25 | 27.1114 | 410.43 | 3.29 | 13.60 |
| 26 | 27.6234 | 854.52 | 3.23 | 28.31 |
| 27 | 29.0481 | 141.10 | 3.07 | 4.67 |
| 28 | 29.8500 | 542.88 | 2.99 | 17.99 |
| 29 | 30.9135 | 172.48 | 2.89 | 5.71 |
| 30 | 32.3447 | 97.54 | 2.77 | 3.23 |
| 31 | 32.6808 | 107.16 | 2.74 | 3.55 |
| 32 | 34.6734 | 176.87 | 2.59 | 5.86 |
| 33 | 36.5832 | 139.35 | 2.46 | 4.62 |
| 34 | 37.8560 | 117.14 | 2.38 | 3.88 |
| 35 | 39.3798 | 114.32 | 2.29 | 3.79 |

In some embodiments of the present disclosure, the salt of compound (I) is a compound of formula (III-1),

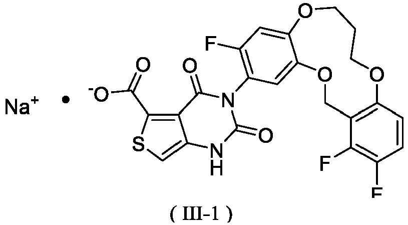

The present disclosure also provides a crystal form S2 of the compound of formula (III-1), wherein the crystal form S2 has an X-ray powder diffraction pattern obtained using Cu Kα radiation with characteristic diffraction peaks at 20 angles of 5.20±0.20°, 7.63±0.20°, 16.00±0.20°, 19.53±0.20°, and 20.97±0.20°.

In some embodiments of the present disclosure, the above crystal form S2 has an X-ray powder diffraction pattern obtained using Cu Kα radiation with characteristic diffraction peaks at 2θ angles of 5.20°, 16.00°, 20.97°, 19.53°, 7.63°, and 12.65°.

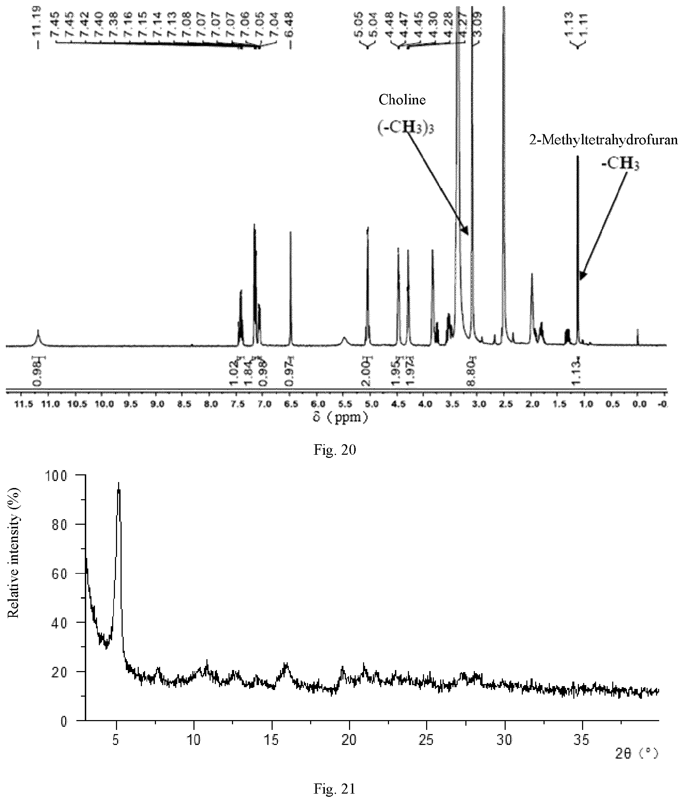

In some embodiments of the present disclosure, the XRPD pattern of the above crystal form S2 obtained using Cu Kα radiation is substantially as shown in Fig. 21.

In some embodiments of the present disclosure, in the XRPD pattern of the above crystal form S2 obtained using Cu Kα radiation, data of the diffraction peaks are as shown in Table 10:

**Table 10 XRPD data of the crystal form S2 of the compound of formula (III-1)**

| **No.** | 2θ[°] | Peak height [cts] | D-spacing [Å] | Relative intensity [%] |
|---|---|---|---|---|
| 1 | 5.2025 | 847.30 | 16.99 | 100.00 |
| 2 | 7.6322 | 59.65 | 11.58 | 7.04 |
| 3 | 12.6484 | 55.54 | 7.00 | 6.56 |
| 4 | 16.0019 | 115.54 | 5.54 | 13.64 |
| 5 | 19.5290 | 88.58 | 4.55 | 10.45 |
| 6 | 20.9731 | 100.61 | 4.24 | 11.87 |

The present disclosure also provides a crystal form S3 of the compound of formula (III-1), wherein the crystal form S3 has an X-ray powder diffraction pattern obtained using Cu Kα radiation with characteristic diffraction peaks at 20 angles of 4.36±0.20°, 8.45±0.20°, 11.94±0.20°, 21.94±0.20°, and 23.41±0.20°.

In some embodiments of the present disclosure, the above crystal form S3 has an X-ray powder diffraction pattern obtained using Cu Kα radiation with characteristic diffraction peaks at 20 angles of 4.36°, 8.45°, 11.94°, 21.94°, and 23.41°.

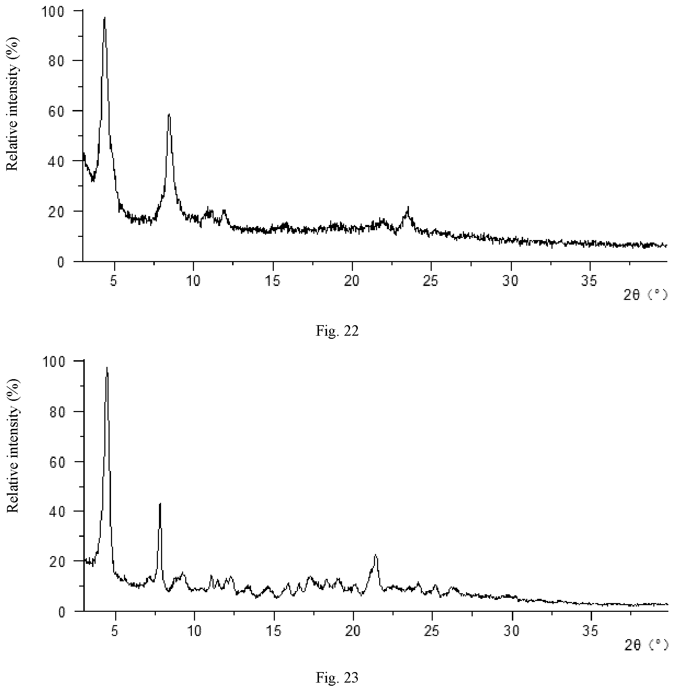

In some embodiments of the present disclosure, the XRPD pattern of the above crystal form S3 obtained using Cu Kα radiation is substantially as shown in Fig. 22.

In some embodiments of the present disclosure, in the XRPD pattern of the above crystal form S3 obtained using Cu Kα radiation, data of the diffraction peaks are as shown in Table 11:

**Table 11 XRPD data of the crystal form S3 of the compound of formula (III-1)**

| **No.** | 2θ[°] | Peak height [cts] | D-spacing [Å] | Relative intensity [%] |
|---|---|---|---|---|
| 1 | 4.3611 | 1210.06 | 20.26 | 100.00 |
| 2 | 8.4469 | 763.56 | 10.47 | 63.10 |
| 3 | 11.9416 | 116.55 | 7.41 | 9.63 |
| 4 | 21.9435 | 84.37 | 4.05 | 6.97 |
| 5 | 23.4155 | 147.92 | 3.80 | 12.22 |

In some embodiments of the present disclosure, the above compound of formula (IV) is a compound of formula (IV-1),

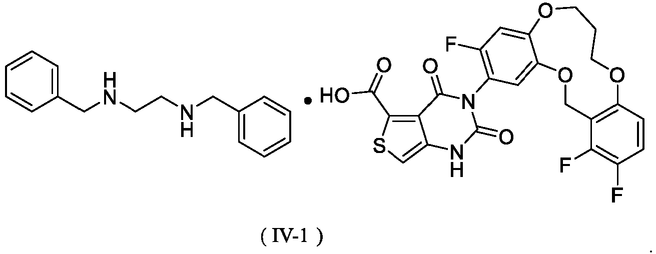

The present disclosure also provides a crystal form S4 of the compound of formula (IV-1), wherein the crystal form S4 has an X-ray powder diffraction pattern obtained using Cu Kα radiation with characteristic diffraction peaks at 20 angles of 4.48±0.20°, 7.81±0.20°, 11.07±0.20°, 12.32±0.20°, 17.23±0.20°, 18.30±0.20°, 19.02±0.20°, and 21.46±0.20°.

In some embodiments of the present disclosure, the above crystal form S4 has an X-ray powder diffraction pattern obtained using Cu Kα radiation with characteristic diffraction peaks at 20 angles of 4.48±0.20°, 7.81±0.20°, 9.30±0.20°, 11.07±0.20°, 12.32±0.20°, 16.62±0.20°, 17.23±0.20°, 18.30±0.20°, 19.02±0.20°, 21.46±0.20°, 24.10±0.20°, and 25.19±0.20°.

In some embodiments of the present disclosure, the above crystal form S4 has an X-ray powder diffraction pattern obtained using Cu Kα radiation with characteristic diffraction peaks at 20 angles of 4.48°, 7.81°, 9.30°, 11.07°, 11.44°, 12.32°, 13.40°, 14.63°, 15.92°, 16.62°, 17.23°, 18.30°, 19.02°, 20.10°, 21.46°, 24.10°, 25.19°, 26.24°, and 29.80°.

In some embodiments of the present disclosure, the XRPD pattern of the above crystal form S4 obtained using Cu Kα radiation is substantially as shown in Fig. 23.

In some embodiments of the present disclosure, in the XRPD pattern of the above crystal form S4 obtained using Cu Kα radiation, data of the diffraction peaks are as shown in Table 12:

**Table 12 XRPD data of the crystal form S4 of the compound of formula (IV-1)**

| **No.** | 2θ[°] | Peak height [cts] | D-spacing [Å] | Relative intensity [%] |
|---|---|---|---|---|
| 1 | 4.4848 | 3600.21 | 19.70 | 100.00 |
| 2 | 7.8063 | 1494.35 | 11.33 | 41.51 |
| 3 | 9.3008 | 271 | 9.51 | 7.53 |
| 4 | 11.0678 | 300.31 | 7.99 | 8.34 |
| 5 | 11.4444 | 231.12 | 7.73 | 6.42 |
| 6 | 12.3177 | 308.31 | 7.19 | 8.56 |
| 7 | 13.3987 | 152.98 | 6.61 | 4.25 |
| 8 | 14.6349 | 160.51 | 6.05 | 4.46 |
| 9 | 15.919 | 199.14 | 5.57 | 5.53 |
| 10 | 16.6151 | 206.05 | 5.34 | 5.72 |
| 11 | 17.2262 | 323.98 | 5.15 | 9.00 |
| 12 | 18.2973 | 280.88 | 4.85 | 7.80 |
| 13 | 19.0173 | 285.64 | 4.67 | 7.93 |
| 14 | 20.1025 | 193.31 | 4.42 | 5.37 |
| 15 | 21.4583 | 697.89 | 4.14 | 19.38 |
| 16 | 24.1016 | 226.56 | 3.69 | 6.29 |
| 17 | 25.1925 | 206.54 | 3.54 | 5.74 |
| 18 | 26.2412 | 177.5 | 3.40 | 4.93 |
| 19 | 29.7967 | 69.93 | 3.00 | 1.94 |

In some embodiments of the present disclosure, the above compound of formula (IV) is a compound of formula (IV-2),

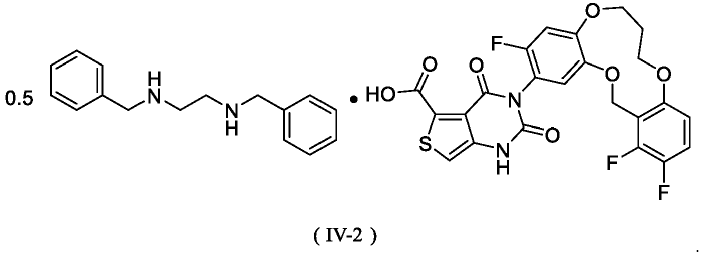

The present disclosure also provides a crystal form S5 of the compound of formula (IV-2), wherein the crystal form S5 has an X-ray powder diffraction pattern obtained using Cu Kα radiation with characteristic diffraction peaks at 2θ angles of 4.76±0.20°, 8.21±0.20°, 9.60±0.20°, 15.81±0.20°, 16.90±0.20°, 22.18±0.20°, 22.98±0.20°, and 25.74±0.20°.

In some embodiments of the present disclosure, the above crystal form S5 has an X-ray powder diffraction pattern obtained using Cu Kα radiation with characteristic diffraction peaks at 20 angles of 4.76±0.20°, 8.21±0.20°, 9.60±0.20°, 15.81±0.20°, 16.90±0.20°, 20.28±0.20°, 21.15±0.20°, 22.18±0.20°, 22.98±0.20°, 23.92±0.20°, 24.93±0.20°, and 25.74±0.20°.

In some embodiments of the present disclosure, the above crystal form S5 has an X-ray powder diffraction pattern obtained using Cu Kα radiation with characteristic diffraction peaks at 20 angles of 4.76°, 8.21°, 9.60°, 11.60°, 13.44°, 14.38°, 15.81°, 16.90°, 19.79°, 20.28°, 21.15°, 22.18°, 22.98°, 23.92°, 24.93°, 25.74°, 28.46°, and 30.72°.

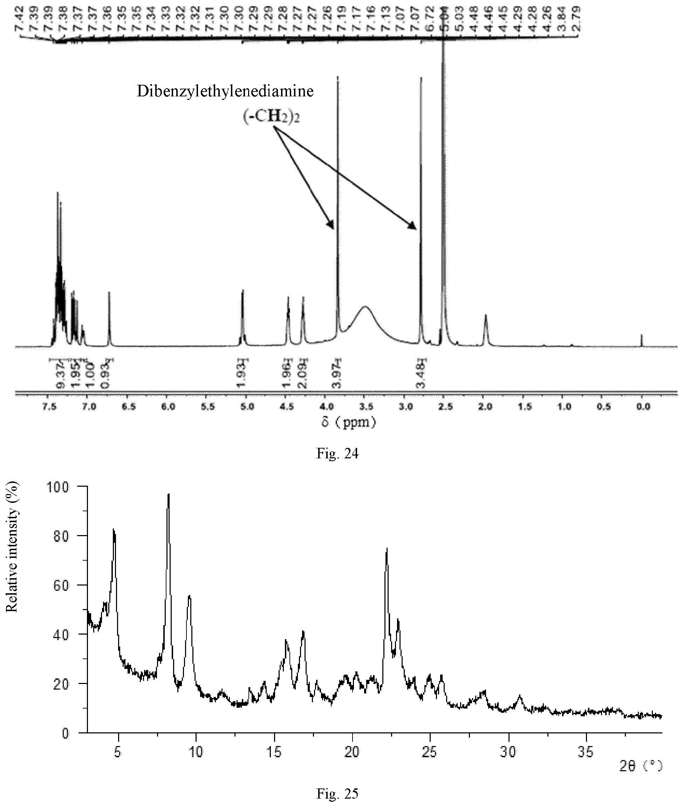

In some embodiments of the present disclosure, the XRPD pattern of the above crystal form S5 obtained using Cu Kα radiation is substantially as shown in Fig. 25.

In some embodiments of the present disclosure, in the XRPD pattern of the above crystal form S5 obtained using Cu Kα radiation, data of the diffraction peaks are as shown in Table 13:

**Table 13 XRPD data of the crystal form S5 of the compound of formula (IV-2)**

| **No.** | 2θ[°] | Peak height [cts] | D-spacing [Å] | Relative intensity [%] |
|---|---|---|---|---|
| 1 | 4.7612 | 832.04 | 18.56 | 59.31 |
| 2 | 8.2088 | 1402.92 | 10.77 | 100.00 |
| 3 | 9.6014 | 686.31 | 9.21 | 48.92 |
| 4 | 11.5996 | 70.65 | 7.63 | 5.04 |
| 5 | 13.4411 | 109.96 | 6.59 | 7.84 |
| 6 | 14.3758 | 152.87 | 6.16 | 10.90 |
| 7 | 15.8116 | 436.78 | 5.60 | 31.13 |
| 8 | 16.9 | 500.42 | 5.25 | 35.67 |
| 9 | 19.7919 | 153.56 | 4.49 | 10.95 |
| 10 | 20.2791 | 200.1 | 4.38 | 14.26 |
| 11 | 21.1453 | 161.47 | 4.20 | 11.51 |
| 12 | 22.182 | 1066.1 | 4.01 | 75.99 |
| 13 | 22.9765 | 563.35 | 3.87 | 40.16 |
| 14 | 23.9179 | 175.3 | 3.72 | 12.50 |
| 15 | 24.926 | 196.32 | 3.57 | 13.99 |
| 16 | 25.7386 | 200.16 | 3.46 | 14.27 |
| 17 | 28.4605 | 127.63 | 3.14 | 9.10 |
| 18 | 30.7247 | 110.78 | 2.91 | 7.90 |

The present disclosure also provides a crystal form A2 of the compound of formula (I), wherein the crystal form A2 has an X-ray powder diffraction pattern obtained using Cu Kα radiation with characteristic diffraction peaks at 20 angles of 6.94±0.20°, 13.39±0.20°, 13.93±0.20°, 17.88±0.20°, 20.41±0.20°, 22.09±0.20°, 24.08±0.20°, and 25.04±0.20°.

In some embodiments of the present disclosure, the above crystal form A2 has an X-ray powder diffraction pattern obtained using Cu Kα radiation with characteristic diffraction peaks at 2θ angles of 6.94°, 13.39°, 13.93°, 17.88°, 20.41°, 22.09°, 24.08°, and 25.04°.

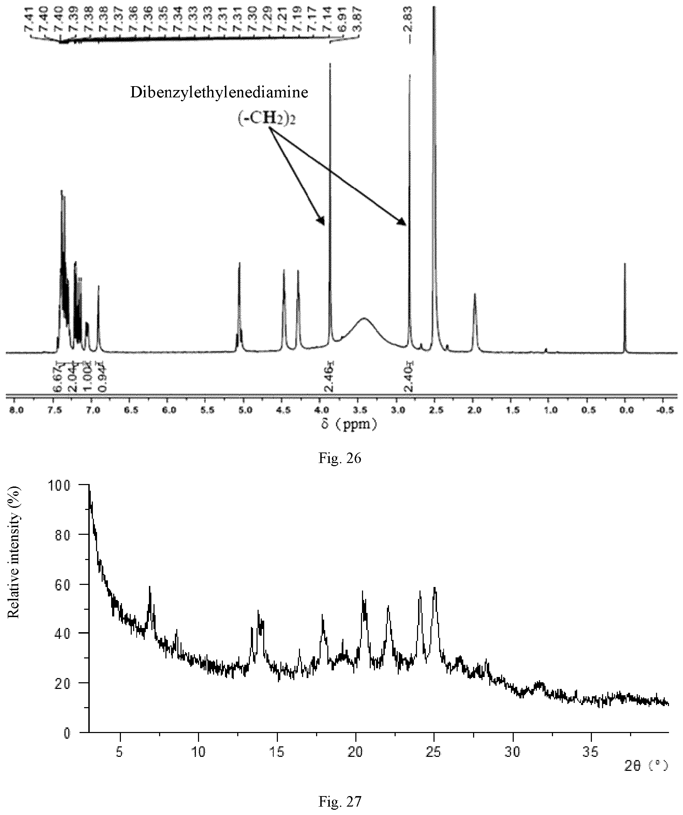

In some embodiments of the present disclosure, the XRPD pattern of the above crystal form A2 obtained using Cu Kα radiation is substantially as shown in Fig. 27.

In some embodiments of the present disclosure, in the XRPD pattern of the above crystal form A2 obtained using Cu Kα radiation, data of the diffraction peaks are as shown in Table 14:

**Table 14 XRPD data of the crystal form A2 of the compound of formula (I)**

| **No.** | 2θ[°] | Peak height [cts] | D-spacing [Å] | Relative intensity [%] |
|---|---|---|---|---|
| 1 | 6.9442 | 102.40 | 12.73 | 30.56 |
| 2 | 13.3861 | 158.54 | 6.61 | 47.32 |
| 3 | 13.9282 | 170.21 | 6.36 | 50.80 |
| 4 | 17.8842 | 182.37 | 4.96 | 54.43 |
| 5 | 20.4147 | 290.80 | 4.35 | 86.79 |
| 6 | 22.0877 | 246.75 | 4.02 | 73.64 |
| 7 | 24.0774 | 325.71 | 3.70 | 97.21 |
| 8 | 25.0406 | 335.05 | 3.56 | 100.00 |
| 9 | 31.7213 | 49.46 | 2.82 | 14.76 |

The present disclosure also provides a crystal form A3 of the compound of formula (I), wherein the crystal form A3 has an X-ray powder diffraction pattern obtained using Cu Kα radiation with characteristic diffraction peaks at 20 angles of 4.47±0.20°, 5.75±0.20°, 8.16±0.20°, 10.04±0.20°, 14.71±0.20°, 15.81±0.20°, 21.67±0.20°, and 23.30±0.20°.

In some embodiments of the present disclosure, the above crystal form A3 has an X-ray powder diffraction pattern obtained using Cu Kα radiation with characteristic diffraction peaks at 20 angles of 4.47°, 5.75°, 8.16°, 10.04°, 14.71°, 15.81°, 21.67°, and 23.30°.

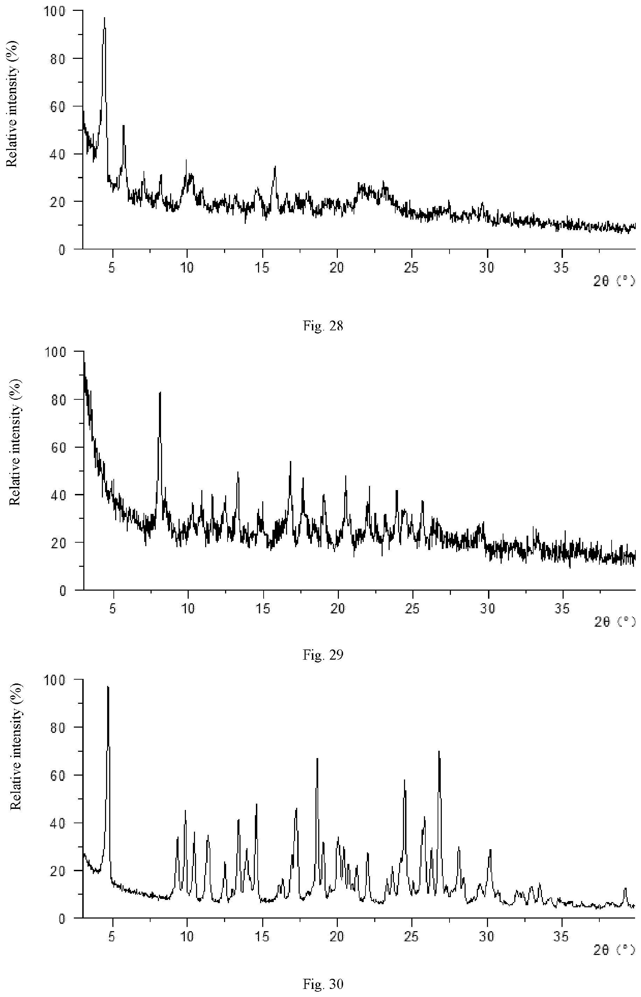

In some embodiments of the present disclosure, the XRPD pattern of the above crystal form A3 obtained using Cu Kα radiation is substantially as shown in Fig. 28.

In some embodiments of the present disclosure, in the XRPD pattern of the above crystal form A3 obtained using Cu Kα radiation, data of the diffraction peaks are as shown in Table 15:

**Table 15 XRPD data of the crystal form A3 of the compound of formula (I)**

| **No.** | 2θ[°] | Peak height [cts] | D-spacing [Å] | Relative intensity [%] |
|---|---|---|---|---|
| 1 | 4.4740 | 407.42 | 19.75 | 100.00 |
| 2 | 5.7545 | 161.36 | 15.36 | 39.60 |
| 3 | 8.1558 | 61.78 | 10.84 | 15.16 |
| 4 | 10.0359 | 73.91 | 8.81 | 18.14 |
| 5 | 14.7076 | 62.07 | 6.02 | 15.23 |
| 6 | 15.8073 | 111.45 | 5.61 | 27.35 |
| 7 | 21.6688 | 65.39 | 4.10 | 16.05 |
| 8 | 23.3020 | 62.14 | 3.82 | 15.25 |

The present disclosure also provides a crystal form A4 of the compound of formula (I), wherein the crystal form A4 has an X-ray powder diffraction pattern obtained using Cu Kα radiation with characteristic diffraction peaks at 20 angles of 8.07±0.20°, 16.82±0.20°, 13.30±0.20°, 19.08±0.20°, 17.66±0.20°, 20.54±0.20°, 21.99±0.20°, and 25.61±0.20°.

In some embodiments of the present disclosure, the above crystal form A4 has an X-ray powder diffraction pattern obtained using Cu Kα radiation with characteristic diffraction peaks at 2θ angles of 8.07°, 10.87°, 12.40°, 13.30°, 16.82°, 17.66°, 19.08°, 20.54°, 21.99°, and 25.61°.

In some embodiments of the present disclosure, the XRPD pattern of the above crystal form A4 obtained using Cu Kα radiation is substantially as shown in Fig. 29.

In some embodiments of the present disclosure, in the XRPD pattern of the above crystal form A4 obtained using Cu Kα radiation, data of the diffraction peaks are as shown in Table 16:

**Table 16 XRPD data of the crystal form A4 of the compound of formula (I)**

| **No.** | 2θ[°] | Peak height [cts] | D-spacing [Å] | Relative intensity [%] |
|---|---|---|---|---|
| 1 | 8.0697 | 224.83 | 10.96 | 100.00 |
| 2 | 10.8676 | 51.41 | 8.14 | 22.87 |
| 3 | 12.3958 | 52.13 | 7.14 | 23.18 |
| 4 | 13.3004 | 102.11 | 6.66 | 45.42 |
| 5 | 16.8219 | 108.08 | 5.27 | 48.07 |
| 6 | 17.6567 | 66.03 | 5.02 | 29.37 |
| 7 | 19.0798 | 71.51 | 4.65 | 31.81 |
| 8 | 20.5443 | 64.31 | 4.32 | 28.61 |
| 9 | 21.9865 | 59.51 | 4.04 | 26.47 |
| 10 | 25.6132 | 54.39 | 3.48 | 24.19 |

The present disclosure also provides a crystal form A5 of the compound of formula (I), wherein the crystal form A5 has an X-ray powder diffraction pattern obtained using Cu Kα radiation with characteristic diffraction peaks at 20 angles of 4.71±0.20°, 9.86±0.20°, 13.43±0.20°, 14.61±0.20°, 17.32±0.20°, 18.66±0.20°, 24.52±0.20°, and 26.82±0.20°.

The present disclosure also provides a crystal form A5 of the compound of formula (I), wherein the crystal form A5 has an X-ray powder diffraction pattern obtained using Cu Kα radiation with characteristic diffraction peaks at 2θ angles of 4.71±0.20°, 9.34±0.20°, 9.86±0.20°, 10.45±0.20°, 11.34±0.20°, 13.43±0.20°, 14.61±0.20°, 17.32±0.20°, 26.82±0.20°, 18.66±0.20°, 24.52±0.20°, and 25.85±0.20°.

In some embodiments of the present disclosure, the above crystal form A5 has an X-ray powder diffraction pattern obtained using Cu Kα radiation with characteristic diffraction peaks at 20 angles of 4.71°, 9.34°, 9.86°, 10.45°, 11.34°, 11.48°, 12.50°, 13.43°, 13.99°, 14.61°, 16.36°, 16.98°, 17.32°, 18.66°, 19.06°, 19.50°, 20.01°, 20.45°, 20.76 °, 21.34°, 22.04°, 23.32°, 23.68°, 24.52°, 25.09°, 25.85°, 26.28°, 26.82°, 28.10°, 28.43°, 29.47°, 30.21°, 30.75°, 31.96°, 32.44°, 32.86°, and 33.49°.

In some embodiments of the present disclosure, the XRPD pattern of the above crystal form A5 obtained using Cu Kα radiation is substantially as shown in Fig. 30.

In some embodiments of the present disclosure, in the XRPD pattern of the above crystal form A5 obtained using Cu Kα radiation, data of the diffraction peaks are as shown in Table 17:

**Table 17 XRPD data of the crystal form A5 of the compound of formula (I)**

| **No.** | 2θ[°] | Peak height [cts] | D-spacing [Å] | Relative intensity [%] |
|---|---|---|---|---|
| 1 | 4.7102 | 2614.63 | 18.76 | 100.00 |
| 2 | 9.3357 | 822.07 | 9.47 | 31.44 |
| 3 | 9.8594 | 1182.94 | 8.97 | 45.24 |
| 4 | 10.4538 | 904.64 | 8.46 | 34.60 |
| 5 | 11.3381 | 880.31 | 7.80 | 33.67 |
| 6 | 11.4807 | 698.67 | 7.71 | 26.72 |
| 7 | 12.4988 | 519.34 | 7.08 | 19.86 |
| 8 | 13.4345 | 1131.24 | 6.59 | 43.27 |
| 9 | 13.9868 | 701.52 | 6.33 | 26.83 |
| 10 | 14.6071 | 1305.99 | 6.06 | 49.95 |
| 11 | 16.3609 | 317.00 | 5.42 | 12.12 |
| 12 | 16.9764 | 603.12 | 5.22 | 23.07 |
| 13 | 17.3211 | 1167.45 | 5.12 | 44.65 |
| 14 | 18.6645 | 1955.56 | 4.75 | 74.79 |
| 15 | 19.0646 | 806.07 | 4.66 | 30.83 |
| 16 | 19.5012 | 205.21 | 4.55 | 7.85 |
| 17 | 20.0057 | 804.70 | 4.44 | 30.78 |
| 18 | 20.4481 | 745.91 | 4.34 | 28.53 |
| 19 | 20.7600 | 515.58 | 4.28 | 19.72 |
| 20 | 21.3403 | 475.96 | 4.16 | 18.20 |
| 21 | 22.0447 | 675.37 | 4.03 | 25.83 |
| 22 | 23.3188 | 294.80 | 3.81 | 11.28 |
| 23 | 23.6770 | 474.69 | 3.76 | 18.16 |
| 24 | 24.5233 | 1630.75 | 3.63 | 62.37 |
| 25 | 25.0865 | 264.04 | 3.55 | 10.10 |
| 26 | 25.8474 | 1114.53 | 3.45 | 42.63 |
| 27 | 26.2826 | 732.58 | 3.39 | 28.02 |
| 28 | 26.8193 | 2022.49 | 3.32 | 77.35 |
| 29 | 28.1015 | 736.51 | 3.18 | 28.17 |
| 30 | 28.4308 | 338.23 | 3.14 | 12.94 |
| 31 | 29.4700 | 214.61 | 3.03 | 8.21 |
| 32 | 30.2107 | 731.78 | 2.96 | 27.99 |
| 33 | 30.7538 | 154.63 | 2.91 | 5.91 |
| 34 | 31.9555 | 175.42 | 2.80 | 6.71 |
| 35 | 32.4416 | 138.18 | 2.76 | 5.28 |
| 36 | 32.8619 | 189.84 | 2.73 | 7.26 |
| 37 | 33.4922 | 308.51 | 2.68 | 11.80 |
| 38 | 34.2690 | 102.71 | 2.62 | 3.93 |
| 39 | 34.7701 | 89.44 | 2.58 | 3.42 |
| 40 | 38.0552 | 53.09 | 2.36 | 2.03 |
| 41 | 39.2586 | 239.71 | 2.29 | 9.17 |

The present disclosure also provides a crystal form A6 of the compound of formula (I), wherein the crystal form A6 has an X-ray powder diffraction pattern obtained using Cu Kα radiation with characteristic diffraction peaks at 20 angles of 6.16±0.20°, 7.28±0.20°, 10.66±0.20°, 14.02±0.20°, 17.37±0.20°, 24.56±0.20°, and 25.76±0.20°.

In some embodiments of the present disclosure, the above crystal form A6 has an X-ray powder diffraction pattern obtained using Cu Kα radiation with characteristic diffraction peaks at 20 angles of 6.16°, 7.28°, 10.66°, 14.02°, 17.37°, 24.56°, and 25.76°.

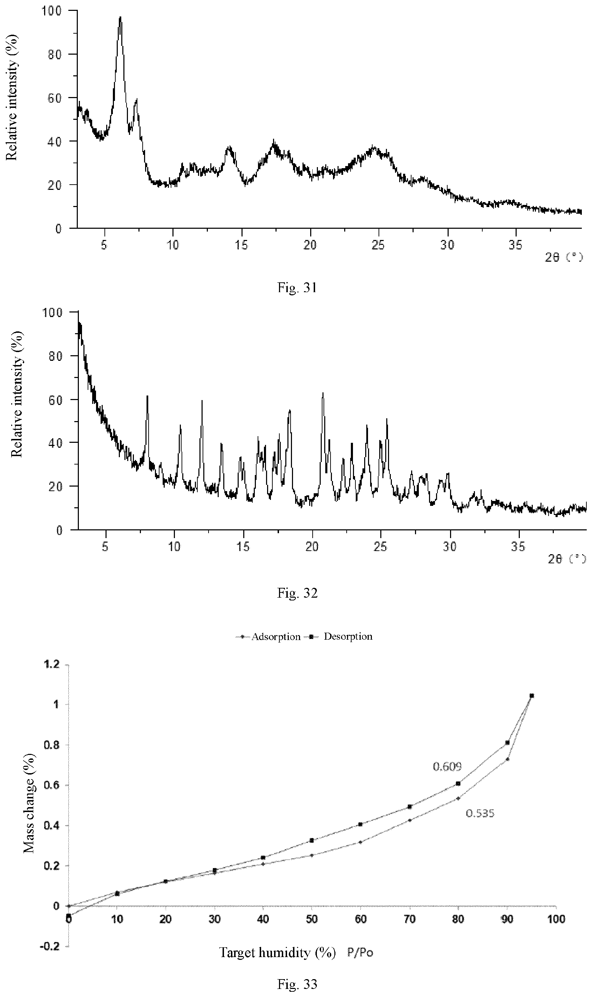

In some embodiments of the present disclosure, the XRPD pattern of the above crystal form A6 obtained using Cu Kα radiation is substantially as shown in Fig. 31.

In some embodiments of the present disclosure, in the XRPD pattern of the above crystal form A6 obtained using Cu Kα radiation, data of the diffraction peaks are as shown in Table 18:

**Table 18 XRPD data of the crystal form A6 of the compound of formula (I)**

| **No.** | 2θ[°] | Peak height [cts] | D-spacing [Å] | Relative intensity [%] |
|---|---|---|---|---|
| 1 | 6.1565 | 1097.30 | 14.36 | 100.00 |
| 2 | 7.2826 | 541.81 | 12.14 | 49.38 |
| 3 | 10.6601 | 137.68 | 8.30 | 12.55 |
| 4 | 14.0199 | 265.72 | 6.32 | 24.22 |
| 5 | 17.3703 | 283.14 | 5.11 | 25.80 |
| 6 | 24.5631 | 256.02 | 3.62 | 23.33 |
| 7 | 25.7559 | 213.62 | 3.46 | 19.47 |

The present disclosure also provides a crystal form A7 of the compound of formula (I), wherein the crystal form A7 has an X-ray powder diffraction pattern obtained using Cu Kα radiation with characteristic diffraction peaks at 2θ angles of 8.03±0.20°, 12.00±0.20°, 16.10±0.20°, 17.58±0.20°, 18.35±0.20°, 20.76±0.20°, 23.98±0.20°, and 25.42±0.20°.

In some embodiments of the present disclosure, the above crystal form A7 has an X-ray powder diffraction pattern obtained using Cu Kα radiation with characteristic diffraction peaks at 20 angles of 8.03±0.20°, 10.41±0.20°, 12.00±0.20°, 16.10±0.20°, 17.58±0.20°, 18.35±0.20°, 20.76±0.20°, 21.23±0.20°, 22.87±0.20°, 23.98±0.20°, 24.98±0.20°, and 25.42±0.20°.

In some embodiments of the present disclosure, the above crystal form A7 has an X-ray powder diffraction pattern obtained using Cu Kα radiation with characteristic diffraction peaks at 2θ angles of 8.03°, 10.41°, 12.00°, 13.44°, 14.74°, 16.10°, 16.60°, 17.23°, 17.58°, 18.35°, 20.76°, 21.23°, 22.25°, 22.87°, 23.98°, 24.98°, 25.42°, 27.16°, 29.39°, 29.81°, 31.93°, and 33.38°.

In some embodiments of the present disclosure, the XRPD pattern of the above crystal form A7 obtained using Cu Kα radiation is substantially as shown in Fig. 32.

In some embodiments of the present disclosure, in the XRPD pattern of the above crystal form A7 obtained using Cu Kα radiation, data of the diffraction peaks are as shown in Table 19:

**Table 19 XRPD data of the crystal form A7 of the compound of formula (I)**

| **No.** | 2θ[°] | Peak height [cts] | D-spacing [Å] | Relative intensity [%] |
|---|---|---|---|---|
| 1 | 8.0292 | 360.10 | 11.01 | 72.30 |
| 2 | 10.4086 | 285.67 | 8.50 | 57.36 |
| 3 | 11.9958 | 380.33 | 7.38 | 76.36 |
| 4 | 13.4379 | 215.40 | 6.59 | 43.25 |
| 5 | 14.7424 | 184.96 | 6.01 | 37.14 |
| 6 | 16.0950 | 299.65 | 5.51 | 60.16 |
| 7 | 16.5997 | 226.70 | 5.34 | 45.52 |
| 8 | 17.2346 | 217.48 | 5.15 | 43.67 |
| 9 | 17.5828 | 324.57 | 5.04 | 65.17 |
| 10 | 18.3525 | 428.76 | 4.83 | 86.09 |
| 11 | 20.7636 | 498.05 | 4.28 | 100.00 |
| 12 | 21.2299 | 288.49 | 4.19 | 57.92 |
| 13 | 22.2528 | 196.77 | 4.00 | 39.51 |
| 14 | 22.8656 | 270.83 | 3.89 | 54.38 |
| 15 | 23.9767 | 354.61 | 3.71 | 71.20 |
| 16 | 24.9795 | 269.71 | 3.56 | 54.15 |
| 17 | 25.4153 | 383.64 | 3.50 | 77.03 |
| 18 | 27.1616 | 136.78 | 3.28 | 27.46 |
| 19 | 29.3934 | 120.69 | 3.04 | 24.23 |
| 20 | 29.8097 | 160.33 | 3.00 | 32.19 |
| 21 | 31.9326 | 43.61 | 2.80 | 8.76 |
| 22 | 33.3754 | 41.12 | 2.68 | 8.26 |

In some embodiments of the present disclosure, the above crystal form G, crystal form H, crystal form A1, crystal form A2, crystal form A3, crystal form A4, crystal form A5, crystal form A6, crystal form A7, crystal form S1, crystal form S2, crystal form S3, crystal form S4 or crystal form S5 can be in the form of a non-solvate or in the form of a solvate, such as in the form of a hydrate, an organic solvate, or a combination of an organic solvate and a hydrate.

In some embodiments of the present disclosure, the organic solvent of the above organic solvate is selected from ethyl acetate, n-hexane, cyclohexane, n-heptane, dimethyl sulfoxide, methyl tert-butyl ether, tetrahydrofuran, methanol, ethanol, isopropyl alcohol, acetonitrile, acetone and N-methylpyrrolidone.

In some embodiments of the present disclosure, the above crystal form G is a hydrate, and the hydration coefficient thereof is 0 to 5.0. In some embodiments of the present disclosure, the above crystal form H is a hydrate, and the hydration coefficient thereof is 0 to 5.0.

In some embodiments of the present disclosure, the above crystal form A1 is an organic solvate, and the organic solvent is selected from ethyl acetate, n-heptane and a mixture of ethyl acetate and n-heptane. In some embodiments of the present disclosure, the above crystal form A2 is a dimethyl sulfoxide solvate. In some embodiments of the present disclosure, the above crystal form A3 is a methyl tert-butyl ether solvate. In some embodiments of the present disclosure, the above crystal form A4 is an acetone solvate. In some embodiments of the present disclosure, the above crystal form A5 is an N-methylpyrrolidone solvate. In some embodiments of the present disclosure, the above crystal form A6 is a methyl tert-butyl ether solvate. In some embodiments of the present disclosure, the above crystal form A7 is a dimethyl sulfoxide solvate.

The present disclosure also provides use of the above crystal form B, crystal form C or crystal form S1 in the manufacture of a GnRH receptor antagonist-related medicament.

In some embodiments of the present disclosure, the above GnRH receptor antagonist-related medicament is a medicament used in the prevention and/or treatment of an endometriosis and/or uterine fibroid-related disease.

### Technical effect

The compounds of the present disclosure have a significant inhibitory effect on human gonadotropin-releasing hormone receptors with high exposure in plasma, low clearance rate, long half-life, and high oral bioavailability, and exhibit excellent pharmacokinetic properties. The preparation processes of the salt types and crystal forms of the present disclosure are simple, and the salt types and crystal forms are stable, less affected by heat, humidity and light, and are easy to make into formulation.

### Definition and description

Unless otherwise specified, the following terms and phrases used herein are intended to have the following meanings. A specific phrase or term should not be considered indefinite or unclear in the absence of a particular definition, but should be understood in the conventional sense. When a trade name appears herein, it is intended to refer to its corresponding commodity or active ingredient thereof.

The intermediate compounds of the present disclosure can be prepared by a variety of synthetic methods well known to those skilled in the art, including the specific embodiments listed below, the embodiments formed by combining the specific embodiments listed below with other chemical synthetic methods, and the equivalent alternative methods well known to those skilled in the art. The alternative embodiments include, but are not limited to, the examples of the present disclosure.

The chemical reactions in the specific embodiments of the present disclosure are completed in a suitable solvent, which must be suitable for the chemical changes of the present disclosure and the reagents and materials required. In order to obtain the compound of the present disclosure, it is sometimes necessary for those skilled in the art to modify or select synthetic steps or reaction schemes based on the existing embodiments.

The present disclosure will be described in detail below through examples, which are not intended to limit the present disclosure in any way.

The structures of the compounds disclosed herein can be confirmed by conventional methods well known to those skilled in the art. If the present disclosure relates to an absolute configuration of a compound, the absolute configuration can be confirmed by conventional techniques in the art, such as single-crystal X-Ray diffraction (SXRD). In the single-crystal X-Ray diffraction (SXRD), the diffraction intensity data of the cultivated single crystal is collected using a Bruker D8 venture diffractometer with a light source of CuKα radiation in a scanning mode of φ/ω scan; after collecting the relevant data, the crystal structure is further analyzed by the direct method (Shelxs97) to confirm the absolute configuration.

The present disclosure uses the following abbreviations: ACN stands for acetonitrile; DMSO stands for dimethyl sulfoxide; N₂: nitrogen; RH: relative humidity; mL: milliliter; L: liter; min: minute; °C: degree Celsius; µm: micrometer; mm: millimeter; µL: microliter; moL/L: mole per liter; mg: milligram; s: second; nm: nanometer; MPa: megapascal; lux: lx; µw/cm²: microwatt per square centimeter; h: hour; Kg: kilogram; nM: nanomole; rpm: revolutions per minute; XRPD stands for X-ray powder diffraction; DSC stands for differential scanning calorimetry; TGA stands for thermogravimetric analysis; ¹H NMR stands for proton nuclear magnetic resonance.

Compounds of the present disclosure are named according to general naming principles in the art or by ChemDraw^{®} software, and commercially available compounds are named with their vendor directory names. All solvents used in the present disclosure are commercially available.

### Instruments and analytical methods

### (1) The X-ray powder diffraction (XRPD) instrument (X-ray powder diffractometer) of the present disclosure

The XRPD instrument and test parameters are shown in Table 20.

**Table 20 XRPD test parameters**

| Model | X'Pert 3 |
|---|---|
| Type of X-ray | Cu, Kα, Kα1 (Å): 1.540598; Kα2 (Å): 1.544426 |
| | Kα2/Kα1 intensity ratio: 0.50 |
| Settings of X-ray tube | 45 kV, 40 mA |
| Divergence slit | 1/8° |
| Scan mode | Continuous |
| Scan range | From 3.0° to 40.0° |
| Scan time per step | 46.7 seconds |
| Step size | 0.0263° |
| Testing time | Approximately 5 minutes |

### (2) The thermogravimetric analysis (TGA) instrument (Thermalgravimetric Analyzer) and the differential scanning calorimetry (DSC) instrument (Differential Scanning Calorimeter) of the present disclosure

The TGA and DSC instruments and test parameters are shown in Table 21.

**Table 21 TGA and DSC test parameters**

| **Parameter** | **TGA** | **DSC** |
|---|---|---|
| Model of instrument | TA Q5000/5500 Thermogravimetric Analyzer | TA 2500 Differential Scanning Calorimeter |
| Method | Linear temperature increase | Linear temperature increase |
| Sample pan | Aluminum pan, uncovered | Aluminum pan, covered/uncovered |
| Temperature range | Room temperature-a set end-point temperature | 25 °C-a set end-point temperature |
| Scan rate (°C/min) | 10 | 10 |
| Protective gas | Nitrogen | Nitrogen |

### (3) Dynamic Vapor Sorption (DVS)

Instrument model: SMS DVS intrinsic plus dynamic vapor sorption instrument. Detailed DVS parameters are as follows:
Temperature: 25 °C;
Protective gas and flow rate: nitrogen, 200 mL/min;
dm/dt=0.002 %/min;
RH (%) test gradient: 10% RH;
Minimum dm/dt balance time: 10 min;
Maximum balance time: 180 min;
Range of RH (%) test gradient: 0% to 95%.

The classification of hygroscopicity evaluation is as follows:

Absorbing enough water and forming a liquid: deliquescence; ΔW% ≥ 15%: very hygroscopic; 15% > ΔW% ≥ 2%: hygroscopic; 2% > ΔW% ≥ 0.2%: slightly hygroscopic; ΔW% < 0.2%: Not or almost not hygroscopic. ΔW% represents the weight increase of a test sample by moisture absorption at 25 ± 1 °C and 80 ± 2% RH.

### (4) Solution Nuclear Magnetic Resonance (Solution NMR)

Solution NMR spectrum was collected on a Bruker 400M NMR instrument using DMSO-d6 as a solvent.

### (5) High performance liquid chromatography/ion chromatography (HPLC/IC) instruments

In the assay, the molar ratio test was performed by Agilent 1260 high performance liquid chromatography system and ion chromatography system. The analysis conditions are shown in Table 22 and Table 23.

**Table 22 Test conditions of HPLC**

| **Liquid chromatography system** | **Agilent 1260 with DAD detector** | |
|---|---|---|
| Chromatography column | Kinetex EVOC18 100A, 150×4.6 mm, 5 µm | |
| Mobile phase | Phase A: 0.0375% trifluoroacetic acid in water; Phase B: 0.01875% trifluoroacetic acid in acetonitrile | |

| | Time (min) | %B |
|---|---|---|
| Gradient | 0.0 | 10 |
| | 20.0 | 80 |
| | 22.0 | 80 |
| | 22.1 | 10 |
| | 26.0 | 10 |
| Running time | 26.0 minutes | |
| Flow rate of the mobile phase | 1 mL/min | |
| Injection volume | 5 µL | |
| Detection wavelength | 220 nm | |
| Column temperature | 50 °C | |
| Diluent | Acetonitrile/water (1:1, volume ratio) | |

**Table 23 Test conditions of ion chromatography**

| **Ion chromatography system** | **ThermoFisher ICS-1100** |
|---|---|
| Chromatography column | Dionex IonPac^{™} CS12A RFIC^{™} 4×250mm Analytical |
| Mobile phase | 20 mM methanesulfonic acid |
| Running time | 7.0 min |
| Flow rate | 1.0 mL/min |
| Injection volume | 25 µL |
| Current | 80 mA |
| Column temperature | 35 °C |

### BRIEF DESCRIPTION OF DRAWINGS

Fig. 1: The XRPD pattern of the crystal form B of the compound of formula (I).
Fig. 2: The DSC curve of the crystal form B of the compound of formula (I).
Fig. 3: The TGA curve of the crystal form B of the compound of formula (I).
Fig. 4: The XRPD pattern of the crystal form C of the compound of formula (I).
Fig. 5: The DSC curve of the crystal form C of the compound of formula (I).
Fig. 6: The TGA curve of the crystal form C of the compound of formula (I).
Fig. 7: The XRPD pattern of the crystal form A1 of the compound of formula (I).
Fig. 8: The XRPD pattern of the crystal form D of the compound of formula (I).
Fig. 9: The DSC curve of the crystal form D of the compound of formula (I).
Fig. 10: The TGA curve of the crystal form D of the compound of formula (I).
Fig. 11: The XRPD pattern of the crystal form E of the compound of formula (I).
Fig. 12: The DSC curve of the crystal form E of the compound of formula (I).
Fig. 13: The TGA curve of the crystal form E of the compound of formula (I).
Fig. 14: The XRPD pattern of the crystal form F of the compound of formula (I).
Fig. 15: The DSC curve of the crystal form F of the compound of formula (I).
Fig. 16: The TGA curve of the crystal form F of the compound of formula (I).
Fig. 17: The XRPD pattern of the crystal form G of the compound of formula (I).
Fig. 18: The XRPD pattern of the crystal form H of the compound of formula (I).
Fig. 19: The XRPD pattern of the crystal form S1 of the compound of formula (II-1).
Fig. 20: The ¹H NMR spectrum of the crystal form S1 of the compound of formula (II-1).
Fig. 21: The XRPD pattern of the crystal form S2 of the compound of formula (III-1).
Fig. 22: The XRPD pattern of the crystal form S3 of the compound of formula (III-1).
Fig. 23: The XRPD pattern of the crystal form S4 of the compound of formula (IV-1).
Fig. 24: The ¹H NMR spectrum of the crystal form S4 of the compound of formula (IV-1).
Fig. 25: The XRPD pattern of the crystal form S5 of the compound of formula (IV-2).
Fig. 26: The ¹H NMR spectrum of the crystal form S5 of the compound of formula (IV-2).
Fig. 27: The XRPD pattern of the crystal form A2 of the compound of formula (I).
Fig. 28: The XRPD pattern of the crystal form A3 of the compound of formula (I).
Fig. 29: The XRPD pattern of the crystal form A4 of the compound of formula (I).
Fig. 30: The XRPD pattern of the crystal form A5 of the compound of formula (I).
Fig. 31: The XRPD pattern of the crystal form A6 of the compound of formula (I).
Fig. 32: The XRPD pattern of the crystal form A7 of the compound of formula (I).
Fig. 33: The DVS curve of the crystal form B of the compound of formula (I).

### DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Reference example 1: intermediate BB-1

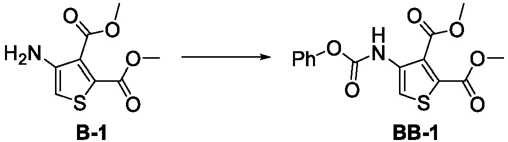

Potassium carbonate (1.65 g, 11.92 mmol) was added to compound **B-1** (2 g, 7.95 mmol, hydrochloride) in tetrahydrofuran (20 mL) and water (10 mL). Phenyl chloroformate (2.49 g, 15.89 mmol) was added dropwise at 5 to 10 °C, and the reaction solution was stirred at 5 to 10 °C for 1 hour. 50 mL of water was added to the reaction solution, and the mixture was extracted twice with ethyl acetate, with 50 mL for each time. The organic phases were combined, washed with 50 mL of saturated brine, dried over anhydrous sodium sulfate and filtered. The filtrate was concentrated to dryness. Ethyl acetate (5 mL) and petroleum ether (50 mL) were added. The mixture was slurried at 30 °C for 30 minutes and filtered. The filter cake was dried under reduced pressure to give compound BB-1. MS-ESI calculated for [M+H]⁺ 336.1, found: 336.1.

### Reference example 2: intermediate BB-2

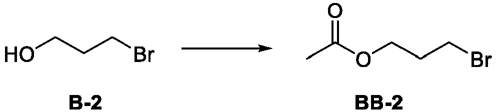

A solution of acetic anhydride (4.04 g, 39.57 mmol) in dichloromethane (5 mL) was added dropwise to a solution of compound **B-2** (3-bromopropanol, 5 g, 35.97 mmol) and 4-dimethylaminopyridine (439.49 mg, 3.6 mmol) in dichloromethane (25 mL). The mixture was warmed to 25 °C and stirred at 25 °C for 4 hours. The reaction solution was washed with 1 mol/L hydrochloric acid (10 mL × 2). The aqueous phase was collected and extracted with dichloromethane (30 mL × 3). The organic phases were combined, washed with saturated aqueous sodium bicarbonate (10 mL × 2), and washed with saturated brine (10 mL × 2). The organic phase was collected, dried over anhydrous sodium sulfate and filtered. The filtrate was concentrated to give an intermediate compound BB-2. ¹H NMR (400 MHz, CDCl₃) δ= 4.18 - 4.25 (m, 2 H), 3.44 - 3.51 (m, 2 H), 2.15 - 2.23 (m, 2 H), 2.07 (s, 3 H).

### Example 1: Preparation of the compound of formula (I)

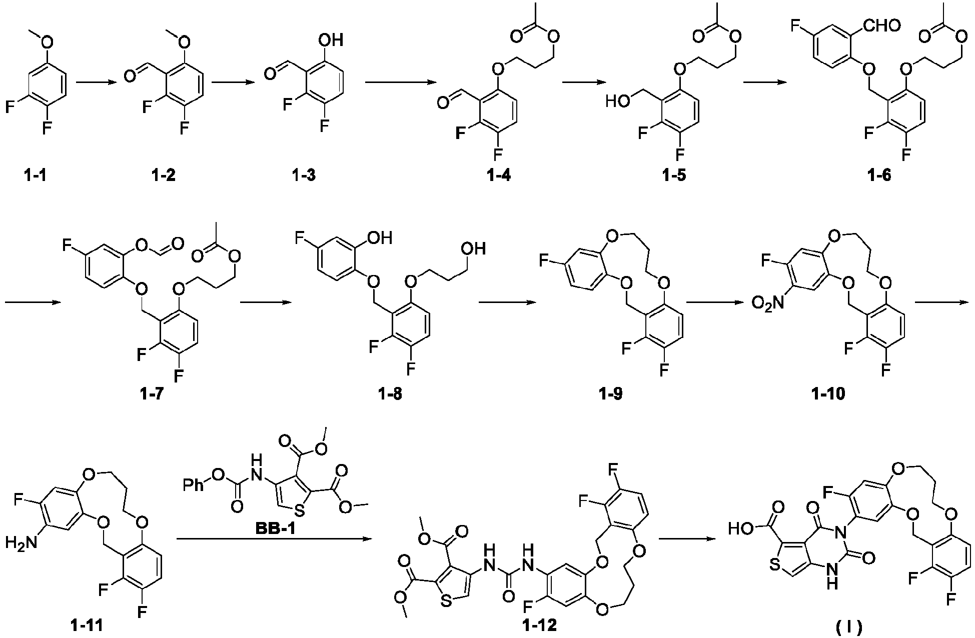

### Step 1

Lithium diisopropylamide (166.53 ml, concentration: 2 mol/L) was added dropwise to a solution of compound 1-1 (3,4-difluoroanisole, 40 g, 277.5 mmol) in tetrahydrofuran (400 mL) at -70 °C, and the reaction system was stirred at -70 °C for 0.5 hours. A solution of N,N-dimethylformamide (25.62 ml, 333.06 mmol) in tetrahydrofuran (24 mL) was added dropwise to the reaction solution at -70 °C to -60 °C, and the reaction system was stirred at -70 °C for 1 hour. Acetic acid (25 mL) and water (100 mL) were added to the reaction system at -65 °C, and the mixture was extracted with ethyl acetate (200 mL × 3). The organic phases were combined, washed with water (100 mL × 3) and saturated brine (100 mL × 3) successively. The organic phase was collected, dried over anhydrous sodium sulfate and filtered. The filtrate was concentrated to give compound 1-2.

### Step 2

Boron tribromide (29.11 g, 116.19 mmol) was added dropwise to a solution of compound 1-2 (10 g, 58.10 mmol) in dichloromethane (100 mL) at -20 °C. The mixture was slowly warmed to 25 °C, and stirred at 25 °C for 12 hours. Methanol (200 mL) and water (100 mL) were added dropwise to the reaction system, and the mixture was heated to 40 °C and stirred at 40 °C for 2 hours. The layers were separated. The aqueous phase was extracted with dichloromethane (300 mL × 2). The organic phases were combined, and extracted with aqueous solution of sodium hydroxide (1 mol/L, 400 mL × 3). The extract was acidified to pH 2 to 3 with concentrated hydrochloric acid. The mixture was extracted with ethyl acetate (300 mL × 3). The organic phase was collected, dried over anhydrous sodium sulfate and filtered. The filtrate was concentrated to give compound **1-3**. ¹H NMR (400 MHz, CDCl₃) δ = 11.13 (s, 1H), 10.29 (s, 1H), 7.37 (q, *J* = 9.3 Hz, 1H), 6.77 - 6.68 (m, 1H).

### Step 3

Sodium iodide (284.42 mg, 1.90 mmol) and potassium carbonate (1.97 g, 14.23 mmol) were added to a solution of compound 1-3 (1.5 g, 9.49 mmol) in N,N-dimethylformamide (20 mL). The mixture was stirred at 25 °C for 0.5 hours. Then, compound **BB-2** (2.06 g, 11.39 mmol) was added, and the mixture was heated to 60 °C and stirred at 60 °C for 12 hours. The reaction system was poured into 30 mL of water, and the mixture was extracted with ethyl acetate (50 mL x 5). The organic phases were combined, washed with water (20 mL x 5), and washed once with saturated brine. The organic phase was dried over anhydrous sodium sulfate and filtered. The filtrate was concentrated to give compound 1-4. ¹H NMR (400 MHz, CDCl₃) δ = 10.45 - 10.38 (m, 1H), 7.37 - 7.28 (m, 1H), 6.74 - 6.65 (m, 1H), 4.28 (t, *J=* 6.2 Hz, 2H), 4.16 - 4.13 (m, 2H), 2.22 - 2.16 (m, 2H), 2.06 (s, 3H).

### Step 4

An aqueous solution (3 mL) of sodium borohydride (490 mg, 12.95 mmol) was added to compound **1-4** (3.25 g, 12.59 mmol) in a solvent of tetrahydrofuran (30 mL) at 0 °C, and the reaction system was stirred at 0 °C for 0.5 hours. Water (10 mL) was added to the reaction system at 0 °C, and the mixture was extracted with ethyl acetate (30 mL × 3). The organic phases were combined, washed with water (10 mL × 2), and washed with saturated brine (10 mL ×). The organic phase was dried over anhydrous sodium sulfate and filtered. The filtrate was concentrated to give compound **1-5.**

### Step 5

Tri-n-butylphosphine (3.71 g, 18.33 mmol) was added to a solution of compound **1-5** (2.65 g, 10.18 mmol) and 5-fluoro-2-hydroxybenzaldehyde (1.57 g, 11.2 mmol) in tetrahydrofuran (20 mL), and the mixture was stirred for 0.1 hours. Then, a solution of 1,1-(azodicarbonyl)dipiperidine (4.62 g, 18.33 mmol) in tetrahydrofuran (5 mL) was added dropwise at 0 °C. The mixture was warmed to 25 °C and stirred at 25 °C for 12 hours. The reaction system was poured into 10 mL of water, and the mixture was extracted with ethyl acetate (30 mL × 3). The organic phases were combined, washed with water (10 mL × 3) and brine (10 mL × 3). The organic phase was collected, dried over anhydrous sodium sulfate, filtered and concentrated. The crude product was purified by column chromatography (silica gel, petroleum ether: ethyl acetate = 1/0 to 20/1) to give compound 1-6. ¹H NMR (400 MHz, CDCl₃) δ = 10.31 (d, *J =* 3.2 Hz, 1H), 7.49 (dd, *J =* 3.2, 8.4 Hz, 1H), 7.33 - 7.27 (m, 1H), 7.23 - 7.18 (m, 1H), 7.18 - 7.11 (m, 1H), 6.72 - 6.57 (m, 1H), 5.25 (d, *J =* 2.8 Hz, 2H), 4.19 (t, *J =* 6.2 Hz, 2H), 4.06 (t, *J =* 6.2 Hz, 2H), 2.09 - 2.06 (m, 2H), 2.04 (s, 3H).

### Step 6

meta-Chloroperoxybenzoic acid (1.66 g, 85% purity, 8.16 mmol) was added to a solution of compound 1-6 (1.04 g, 2.72 mmol) in dichloromethane (10 mL) at 0 °C, and the mixture was warmed to 25 °C and stirred at 25 °C for 12 hours. 2 mL of saturated aqueous solution of sodium sulfite was added to the reaction solution, and then 10 mL of water was added. The mixture was extracted with ethyl acetate (30 mL × 3). The organic phases were combined, washed with water (10 mL × 2), washed with saturated brine (10 mL × 2), dried over anhydrous sodium sulfate and filtered. The filtrate was concentrated. The resulting crude product was purified by preparative chromatography (petroleum ether: ethyl acetate = 3:1) to give compound **1-7.** ¹H NMR (400 MHz, CDCl₃) δ = 8.19 (s, 1H), 7.18 - 7.08 (m, 2H), 7.00 - 6.93 (m, 1H), 6.89 (dd, *J* = 3.2, 8.4 Hz, 1H), 6.63 - 6.57 (m, 1H), 5.14 - 5.09 (m, 2H), 4.23 (t, *J=* 6.2 Hz, 2H), 4.04 (t, *J =* 6.2 Hz, 2H), 2.14 - 2.09 (m, 2H), 2.06 (s, 3H).

### Step 7

An aqueous solution of potassium hydroxide (1 ml, 20% purity, 489.03 µmol) was added to a solution of compound 1-7 (1 g, 2.51 mmol) in methanol (10 mL), and the mixture was stirred at 25 °C for 6 hours. The reaction solution was poured into 10 mL of water, and then the mixture was extracted with ethyl acetate (30 mL × 3). The organic phases were combined, washed with water (10 mL × 2), and washed with saturated brine (10 mL × 2). The organic phase was collected, dried over anhydrous sodium sulfate and filtered. The filtrate was concentrated. The crude product was purified by column chromatography (silica gel, petroleum ether: ethyl acetate = 5/1 to 3/1) to give compound 1-8. ¹H NMR (400 MHz, CDCl₃) δ = 7.15 (q, *J =* 9.2 Hz, 1H), 7.00 (dd, *J =* 5.2, 8.8 Hz, 1H), 6.91 (s, 1H), 6.69 - 6.62 (m, 2H), 6.53 (dt, *J=* 3.0, 8.6 Hz, 1H), 5.17 (d, *J =* 2.0 Hz, 2H), 4.22 (t, *J =* 5.8 Hz, 2H), 3.88 (q, *J=* 5.0 Hz, 2H), 2.49 (br s, 1H), 2.15 - 2.05 (m, 2H).

### Step 8

Sodium hydride (135.04 mg, 60% purity, 3.38 mmol) was added to compound 1-8 (421 mg, 1.28 mmol) in a solvent of tetrahydrofuran (400 mL) at 0 °C, and the mixture was stirred at 0 °C for 0.5 hours. A solution of p-toluenesulfonyl chloride (244.50 mg, 1.28 mmol) in tetrahydrofuran (5 mL) was added dropwise to the reaction system at 0 °C, and the mixture was stirred at 25 °C for 12 hours. 10 mL of water was added to the reaction solution, and the mixture was extracted with ethyl acetate (30 mL × 3). The organic phases were combined, washed with water (10 mL × 2), washed with saturated brine (10 mL × 2), dried over anhydrous sodium sulfate and filtered. The filtrate was concentrated. Then, the crude product was purified by preparative chromatography (petroleum ether: ethyl acetate = 3:1) to give compound 1-9.

### Step 9

Nitric acid (1.46 ml, 60% purity, 19.51 mmol) was added dropwise to a solution of compound 1-9 (53 mg, 170.82 µmol) in acetic acid (1 mL) at 80 °C, and the mixture was stirred at 80 °C for 2 hours. The reaction solution was poured into 40 mL of iced water, and the mixture was adjusted to pH of 7 with saturated aqueous sodium bicarbonate. The aqueous phase was extracted with ethyl acetate (30 mL × 5). The organic phases were combined, washed with water (30 mL × 3), washed with saturated brine (20 mL × 1), dried over anhydrous sodium sulfate and filtered. The filtrate was concentrated to give compound **1-10.** ¹H NMR (400 MHz, CDCl₃) δ = 7.92 (d, *J* = 7.6 Hz, 1H), 7.14 - 7.10 (m, 1H), 6.76 - 6.73 (d, *J =* 12.0 Hz, 1H), 6.57 - 6.12 (m, 1H), 5.20 (d, *J =* 1.2 Hz, 2H), 4.48 - 4.42 (m, 2H), 4.37 - 4.31 (m, 2H), 2.17 - 2.14 (m, 2H).

### Step 10

Wet palladium on carbon (10 mg, 10% purity) was added to a solution of compound **1-10** (46 mg, 129.48 µmol) in ethyl acetate (10 mL), and the system was purged with hydrogen three times. The mixture was stirred at 24 °C for 12 hours under hydrogen (15 psi). The reaction solution was filtered through diatomaceous earth, and the filtrate was concentrated to give compound **1-11.** ESI calculated for [M+H]⁺ 326.1, found 326.1; ¹H NMR (400 MHz, CDCl₃) δ = 7.17 - 7.07 (m, 2H), 6.82 - 6.78 (m, 1H), 6.76 (d, *J* = 11.6 Hz, 1H), 6.68 (d, *J* = 9.0 Hz, 1H), 6.63 - 6.56 (m, 1H), 5.07 (d, *J =* 2.0 Hz, 2H), 4.35 - 4.31 (m, 2H), 4.16 - 4.11 (m, 2H), 2.13 - 2.09 (m, 2H).

### Step 11

Compound BB-1 (26.29 mg, 78.41 µmol) and triethylamine (7.93 mg, 78.41 µmol) were added to a solution of compound **1-11** (41 mg, 78.41 µmol) in tetrahydrofuran (3 mL). The mixture was stirred at 70 °C for 10 hours. The reaction solution was poured into 10 mL of water, and the mixture was extracted with ethyl acetate (30 mL × 5). The organic phases were combined, washed with water (10 mL × 3), washed with saturated brine (10 mL × 1), dried over anhydrous sodium sulfate and filtered. The filtrate was concentrated, and then purified by preparative chromatography (petroleum ether: ethyl acetate = 2/1) to give compound 1-12. MS-ESI calculated for [M+H]⁺ 567.1, found 567.1; ¹H NMR (400 MHz, CDCl₃) δ = 8.86 (s, 1H), 7.97 (s, 1H), 7.81 (d, *J =* 8.0 Hz, 1H), 7.16 - 7.06 (m, 1H), 6.84 (d, *J* = 11.6 Hz, 1H), 6.78 (m, 1H), 6.57 (br s, 1H), 5.16 (d, *J =* 1.6 Hz, 2H), 4.36 (t, *J* = 5.2 Hz, 2H), 4.22 (t, *J =* 5.2 Hz, 2H), 3.91 (s, 3H), 3.90 (s, 3H), 2.11 (m, 2H).

### Step 12

An aqueous solution (1 mL) of lithium hydroxide monohydrate (5.99 mg, 142.85 µmol) was added to a solution of compound **1-12** (22 mg, 28.57 µmol, 73.57% purity) in tetrahydrofuran (2 mL) and methanol (1 mL), and the mixture was stirred at 26 °C for 2 hours. 1 mol/L dilute hydrochloric acid was added to the reaction solution to adjust the pH to approximately 6, and then the mixture was extracted with ethyl acetate (5 mL × 5). The organic phases were combined, and washed with water (5 mL × 3). The organic phase was collected, dried over anhydrous sodium sulfate and filtered. The filtrate was concentrated. The crude product was purified by preparative chromatography (dichloromethane: methanol = 10/1) to give the compound of formula (I). MS-ESI calculated for [M+H]⁺ 521.0, found 521.1.

### Example 2: Preparation of the crystal form A1 of the compound of formula (I)

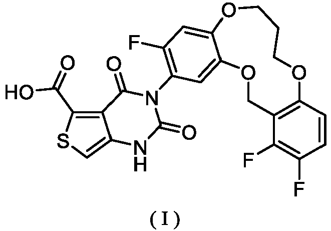

The compound of formula (I) (25 g) was suspended in ethyl acetate (80 mL) at 45 °C to 50 °C, and n-heptane (40 mL) was added under stirring. Then, the mixture was naturally cooled to 15 °C to 20 °C, and stirred for 0.5 hours. The mixture was filtered. The filter cake was washed with a mixed solvent of ethyl acetate (20 mL) and n-heptane (10 mL), and then washed with n-heptane (20 mL). The filter cake was collected, and dried under vacuum to give a solid. The solid was determined by XRPD to be the crystal form A1 of the compound of formula (I). The XRPD pattern is shown in Fig. 7. MS-ESI calculated for [M+H]⁺ 521.0, found 521.1; ¹H NMR (400MHz, DMSO-d6) δ = 14.54 (br s, 1H), 12.02 (s, 1H), 7.46 - 7.34 (m, 2H), 7.26 (d, J=7.8 Hz, 1H), 7.18 (d, J=11.6 Hz, 1H), 7.05 (br dd, J=2.0, 9.4 Hz, 1H), 5.17 - 5.01 (m, 2H), 4.48 (br t, J=4.8 Hz, 2H), 4.30 (br t, J=4.6 Hz, 2H), 1.97 (br s, 2H).

### Example 3: Preparation of the crystal form B of the compound of formula (I)

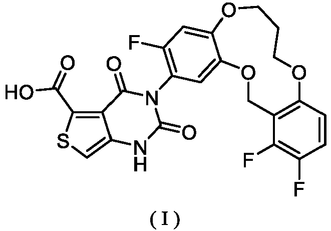

Method 1: The crystal form A1 of the compound of formula (I) (1 g) was weighed, and suspended with stirring in water (10 mL) at 90 °C for 14 hours. Then, the mixture was filtered. The filter cake was dried at 50 °C under reduced pressure to give a solid. The solid was determined by XRPD to be the crystal form B of the compound of formula (I). The XRPD pattern is shown in Fig. 1. The DSC curve is shown in Fig. 2. The TGA curve is shown in Fig. 3.

Method 2: The crystal form A1 of the compound of formula (I) (0.3 g) was weighed, and suspended with stirring in a mixed solvent of acetonitrile (1 mL) and water (5 mL) at 70 °C for 12 hours. Then, the mixture was filtered. The filter cake was dried at 50 °C under reduced pressure to give a solid. The solid was determined by XRPD to be the crystal form B of the compound of formula (I).

### Example 4: Preparation of the crystal form C of the compound of formula (I)

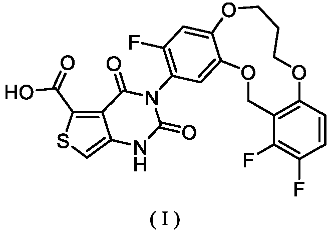

The crystal form A1 of the compound of formula (I) (24.8 mg) was weighed, and suspended with stirring in acetonitrile (0.5 mL) at room temperature (25±3 °C) for 3 days. Then, the mixture was centrifugated to give a wet sample. The wet sample was air-dried at room temperature to give a solid. The solid was determined by XRPD to be the crystal form C of the compound of formula (I). The XRPD pattern is shown in Fig. 4. The DSC curve is shown in Fig. 5. The TGA curve is shown in Fig. 6.

### Example 5: Preparation of the crystal form D of the compound of formula (I)

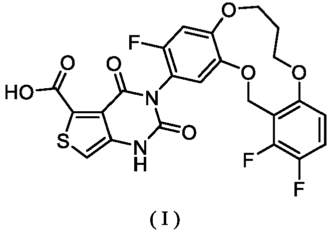

The crystal form A1 of the compound of formula (I) (24.5 mg) was weighed, and dichloromethane (0.5 mL) was added. The mixture was suspended with stirring at room temperature (25±3 °C) for 3 days. Then, the mixture was centrifugated to give a wet sample. The wet sample was air-dried at room temperature to give a solid. The solid was determined by XRPD to be the crystal form D of the compound of formula (I). The XRPD pattern is shown in Fig. 8. The DSC curve is shown in Fig. 9. The TGA curve is shown in Fig. 10.

### Example 6: Preparation of the crystal form E of the compound of formula (I)

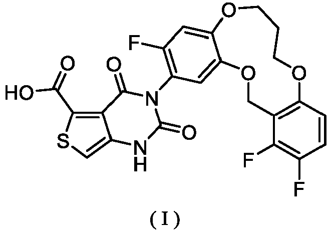

The crystal form A1 of the compound of formula (I) (25.1 mg) was weighed, and suspended with stirring in 0.5 mL of tetrahydrofuran/n-heptane (volume ratio of 1:4) at room temperature (25±3 °C) for 3 days. Then, the mixture was centrifugated to give a wet sample. The wet sample was air-dried at room temperature to give a solid. The solid was determined by XRPD to be the crystal form E of the compound of formula (I). The XRPD pattern is shown in Fig. 11. The DSC curve is shown in Fig. 12. The TGA curve is shown in Fig. 13.

### Example 7: Preparation of the crystal form F of the compound of formula (I)

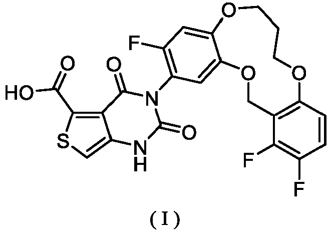

The crystal form A1 of the compound of formula (I) (52.3 mg) was weighed, and suspended with stirring in 0.75 mL of dimethyl sulfoxide/water (volume ratio of 1:4) at room temperature (25±3 °C) for 5 days. Then, the mixture was centrifugated to give a wet sample. The wet sample was air-dried at room temperature to give a solid. Then, the solid was heated to 180 °C, and cooled to room temperature to give a solid. The solid was determined by XRPD to be the crystal form F of the compound of formula (I). The XRPD pattern is shown in Fig. 14. The DSC curve is shown in Fig. 15. The TGA curve is shown in Fig. 16.

### Example 8: Preparation of the crystal form G of the compound of formula (I)

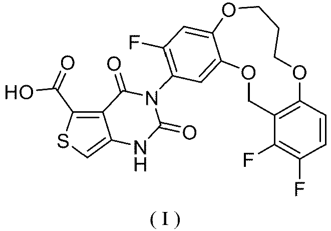

The crystal form A1 of the compound of formula (I) (24.4 mg) was weighed, and suspended with stirring in 0.5 mL of methanol/water (volume ratio of 69:31) at room temperature (25±3 °C) for 3 days. Then, the mixture was centrifugated to give a wet sample. The wet sample was air-dried at room temperature to give a solid. The solid was determined by XRPD to be the crystal form G of the compound of formula (I). The XRPD pattern is shown in Fig. 17.

### Example 9: Preparation of the crystal form H of the compound of formula (I)

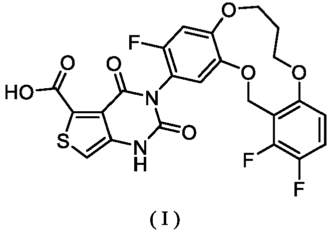

The crystal form G of the compound of formula (I) was heated to 180 °C, and then cooled to room temperature to give a solid. The solid was determined by XRPD to be the crystal form H of the compound of formula (I). The XRPD pattern is shown in Fig. 18.

### Example 10: Preparation of the crystal form S1 of the compound of formula (II)

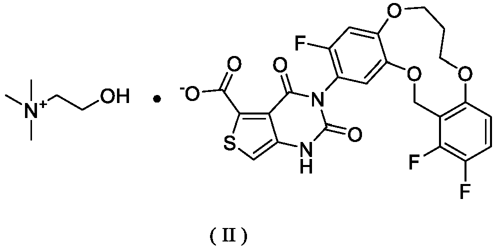

The crystal form A1 of the compound of formula (I) (25.0 mg) and 11.7 mg of choline were suspended with stirring in 2-methyltetrahydrofuran (0.5 mL) at room temperature (25±3 °C) for 3 days. The mixture was transferred to 5 °C and stirred for 1 day. The mixture was then transferred to -20 °C and stirred for 2 days. The mixture was centrifugated, and then dried under vacuum at room temperature to give a solid. The solid was determined by XRPD to be the crystal form S1 of the compound of formula (II). ¹H NMR showed that the molar ratio of choline to the compound of formula (I) was 1.0. The XRPD pattern is shown in Fig. 19. The ¹H NMR spectrum is shown in Fig. 20.

### Example 11: Preparation of the crystal form S2 of the compound of formula (III)

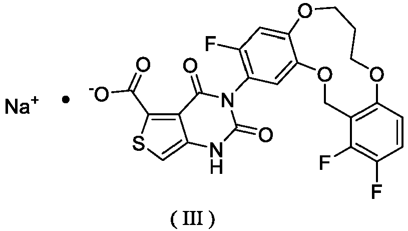

The crystal form A1 of the compound of formula (I) (25.0 mg) and 2.0 mg of sodium hydroxide were suspended with stirring in acetonitrile (0.5 mL) at room temperature (25±3 °C) for 4 days. The mixture was centrifugated, and then dried under vacuum at room temperature to give a solid. The solid was determined by XRPD to be the crystal form S2 of the compound of formula (III). HPLC/IC showed that the molar ratio of base to acid was 1.0. The XRPD pattern is shown in Fig. 21.

### Example 12: Preparation of the crystal form S3 of the compound of formula (III)

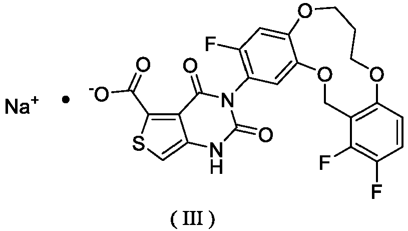

The crystal form A1 of the compound of formula (I) (25.1 mg) and 2.0 mg of sodium hydroxide were suspended with stirring in tetrahydrofuran (0.5 mL) at room temperature (25±3 °C) for 4 days. The mixture was centrifugated, and then dried under vacuum at room temperature to give a solid. The solid was determined by XRPD to be the crystal form S3 of the compound of formula (III). HPLC/IC showed that the molar ratio of base to acid was 1.2. The XRPD pattern is shown in Fig. 22.

### Example 13: Preparation of the crystal form S4 of the compound of formula (IV-1)

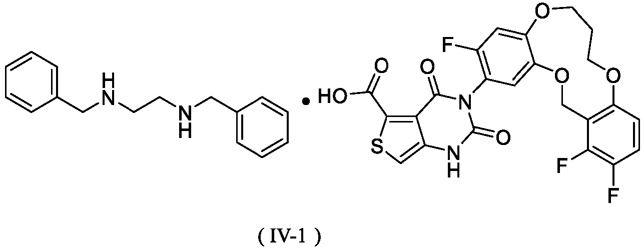

The crystal form A1 of the compound of formula (I) (25.1 mg) and 11.5 mg of dibenzylethylenediamine were suspended with stirring in isopropyl alcohol (0.5 mL) at room temperature (25±3 °C) for 3 days. The mixture was centrifugated, and then dried under vacuum at room temperature to give a solid. The solid was determined by XRPD to be the crystal form S4 of the compound of formula (IV-1). ¹H NMR showed that the molar ratio of dibenzylethylenediamine to the compound of formula (I) was 0.9. The XRPD pattern is shown in Fig. 23. The ¹H NMR spectrum is shown in Fig. 24.

### Example 14: Preparation of the crystal form S5 of the compound of formula (IV-2)

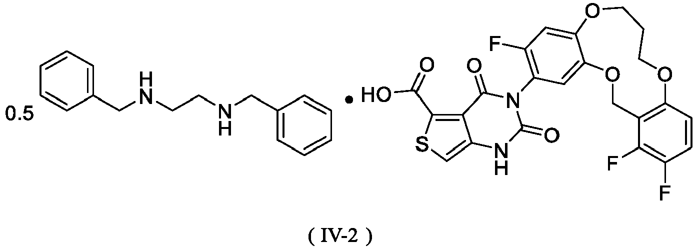

The crystal form A1 of the compound of formula (I) (25.0 mg) and 11.5 mg of dibenzylethylenediamine were suspended with stirring in acetonitrile (0.5 mL) at room temperature (25±3 °C) for 3 days. The mixture was centrifugated, and then dried under vacuum at room temperature to give a solid. The solid was determined by XRPD to be the crystal form S5 of the compound of formula (IV-2). ¹H NMR showed that the molar ratio of dibenzylethylenediamine to the compound of formula (I) was 0.6. The XRPD pattern is shown in Fig. 25. The ¹H NMR spectrum is shown in Fig. 26.

### Example 15: Preparation of the crystal form A2 of the compound of formula (I)

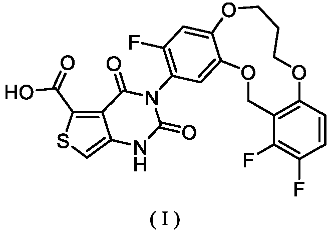

The crystal form A1 of the compound of formula (I) (24.7 g) was weighed, and suspended with stirring in 0.5 mL of dimethyl sulfoxide/water (volume ratio of 1:4) at room temperature (25±3 °C) for 3 days. Then, the mixture was centrifugated to give a wet sample. The wet sample was air-dried at room temperature to give a solid. The solid was determined by XRPD to be the crystal form A2 of the compound of formula (I). The XRPD pattern is shown in Fig. 27.

### Example 16: Preparation of the crystal form A3 of the compound of formula (I)

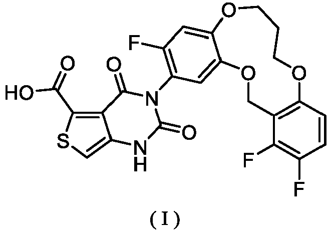

The crystal form A1 of the compound of formula (I) (25.1 mg) was weighed, and dissolved in 0.2 mL of tetrahydrofuran. 1.5 mL of methyl tert-butyl ether was added. Then, the mixture was centrifugated to give a wet sample. The wet sample was air-dried at room temperature to give a solid. The solid was determined by XRPD to be the crystal form **A3** of the compound of formula (I). The XRPD pattern is shown in Fig. 28.

### Example 17: Preparation of the crystal form A4 of the compound of formula (I)

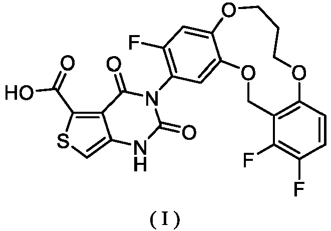

The crystal form A1 of the compound of formula (I) (24.6 mg) was weighed, and dissolved in 0.2 mL of acetone. 1.5 mL of n-heptane was added, and the mixture was stirred at 5 °C for 1 day. The mixture was transferred to -20 °C and stirred for 2 days. Then, the mixture was centrifugated to give a wet sample. The wet sample was air-dried at room temperature to give a solid. The solid was determined by XRPD to be the crystal form **A4** of the compound of formula (I). The XRPD pattern is shown in Fig. 29.

### Example 18: Preparation of the crystal form A5 of the compound of formula (I)

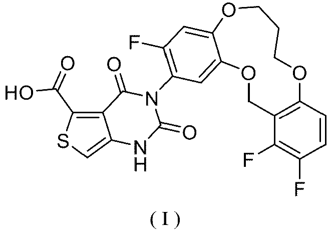

The crystal form A1 of the compound of formula (I) (25.2 mg) was weighed and dissolved in 0.2 mL of N-methylpyrrolidone. 1.0 mL of water was added. Then, the mixture was centrifugated to give a wet sample. The wet sample was air-dried at room temperature to give a solid. The solid was determined by XRPD to be the crystal form A5 of the compound of formula (I). The XRPD pattern is shown in Fig. 30.

### Example 19: Preparation of the crystal form A6 of the compound of formula (I)

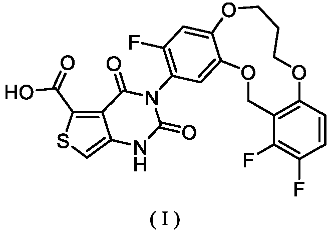

The crystal form A1 of the compound of formula (I) (25.6 mg) was weighed, and suspended with stirring in 0.5 mL of methanol/methyl tert-butyl ether (volume ratio of 1:4) at room temperature (25±3 °C) for 3 days. Then, the mixture was centrifugated to give a wet sample. The wet sample was air-dried at room temperature to give a solid. The solid was determined by XRPD to be the crystal form **A6** of the compound of formula (I). The XRPD pattern is shown in Fig. 31.

### Example 20: Preparation of the crystal form A7 of the compound of formula (I)

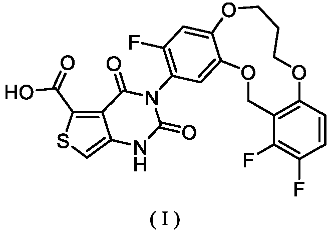

The crystal form A1 of the compound of formula (I) was weighed (52.3 mg), suspended with stirring in 0.75 mL of dimethyl sulfoxide/water (volume ratio of 1:4) at room temperature (25±3 °C) for 5 days. Then, the mixture was centrifugated to give a wet sample. The wet sample was air-dried at room temperature to give a solid. The solid was determined by XRPD to be the crystal form **A7** of the compound of formula (I). The XRPD pattern is shown in Fig. 32.

### Example 21: Solid stability testing of the crystal form B of the compound of formula (I)

According to the "Guidelines for the Stability Testing of Drug Substances and Preparations" (Chinese Pharmacopoeia 2020 Edition Part IV General Rules 9001), in order to evaluate the solid stability of the crystal form B of the compound of formula (I), the crystal form B was investigated for stability under influencing factors (high temperature, high humidity and light), accelerated (40 °C/75% RH), and long-term (25 °C/60% RH) conditions. Influencing factor testing: unless otherwise specified, each sample was placed in an open weighing bottle, which was placed in the corresponding storage container to investigate the stability at the time points of 5 and 10 days. The photostability testing complied with the requirements of ICH Q1B: the illuminated sample was exposed to visible light and ultraviolet light; storage conditions: the sample was illuminated at 5000±500 lux (visible light) and 90 µw/cm² (UV) for 5 days and 10 days. The total illumination received by the sample after 10 days was not less than 1.2×10⁶ Lux·h, and the near-ultraviolet energy was not less than 200 w·h/m². The illuminated sample was placed in a clean weighing bottle, and spread out into a single layer without being covered by anything. The weighing bottle was uncovered and placed in an illumination box for illumination. The control sample was packaged in the same way as the illuminated sample, but the weighing bottle was covered with aluminum film. Long-term and accelerated testing: each sample was placed in a double-layer LDPE bag, respectively, and each layer of the LDPE bag was sealed with a buckle. Then, the sample was placed in an aluminum foil bag, which was heat-sealed. The sample was placed for 1, 2 and 3 months in the accelerated testing, and placed for 3 months under the long-term condition. XRPD analysis was performed on all the stability samples to test the changes in the crystal forms.

25 mg of a sample was taken, weighed accurately, and placed in a 10 mL volumetric flask. 6 mL of acetonitrile was added and the sample was dissolved by ultrasound. The mixture was cooled to room temperature, and water was added to the scale marker. The mixture was shaken well, and the sample was dissolved to give a solution with a concentration of approximately 2.5 mg/mL. The liquid phase was used for sample injection analysis. The test results were compared with the initial test results on day 0. The specific test results are shown in Table 24 below. The HPLC instrument and analysis conditions are shown in Table 25.

**Table 24 Results of solid stability testing of the crystal form B of the compound of formula (I)**

| **Test conditions** | **Conditions of point selection** | **Purity(%)** | **Crystal form** |
|---|---|---|---|
| - | 0 day | 99.4 | Crystal form B |
| High temperature (60 °C, uncovered) | 10 days | 99.4 | Crystal form B |
| High humidity (25 °C, 92.5% relative humidity, uncovered) | 10 days | 99.4 | Crystal form B |
| Visible light + UV (ICH conditions) | The total illumination of visible light reaching 1200000 Lux·h | 99.4 | Crystal form B |
| | The total illumination of UV reaching 200 | | |
| | w·h/m² | | |
| Light-shielding control group | Placed with visible light + UV light, and wrapped with tin foil | 99.4 | Crystal form B |
| Accelerated (40 °C, 75% relative humidity, uncovered) | 1 month | 99.4 | Crystal form B |
| | 2 months | 99.4 | Crystal form B |
| | 3 months | 99.4 | Crystal form B |
| Long term (25 °C, 60% relative humidity, uncovered) | 3 months | 99.4 | Crystal form B |

**Table 25 HPLC instrument information and analysis method**

| Parameter | Settings | | |
|---|---|---|---|
| Chromatography column | Agilent ZORBAX SB C18, **4.6mm×150mm, 3.5µm** | | |
| Mobile phase | Mobile phase A: 0.1% trifluoroacetic acid in water (volume ratio); B: 0.1% trifluoroacetic acid in acetonitrile (volume ratio) | | |
| Gradient | Time (min) | Mobile phase A (%) | Mobile phase B (%) |
| | 0.00 | 90 | 10 |
| | 2.00 | 90 | 10 |
| | 17.00 | 10 | 90 |
| | 22.00 | 10 | 90 |
| | 22.1 | 90 | 10 |
| | 30.00 | 90 | 10 |
| Flow rate | 1.0 mL/min | | |
| Injection volume | 3 µL | | |
| Detection wavelength | 220 nm | | |
| Column temperature | 35 °C | | |

Conclusion: The purity and crystalline form of the crystal form B of the compound of formula (I) did not change significantly under all the stability conditions (high temperature, high humidity, illumination, long-term and accelerated), and the crystal form B of the compound of formula (I) had good chemical stability.

### Example 22: Hygroscopicity study of the crystal form B of the compound of formula (I)

The test was performed using SMS DVS intrinsic plus dynamic vapor sorption instrument. The crystal form B of the compound of formula (I) (10 to 20 mg) was taken and placed in a DVS sample pan for testing.

Test results: The DVS curve of the crystal form B of the compound of formula (I) is shown in Fig. 33, and ΔW% was 0.535%.

Test conclusion: The vapor adsorption of the crystal form B of the compound of formula (I) at 25 °C/80%RH was 2% > ΔW% ≥ 0.2%, indicating that the crystal form B of the compound of formula (I) was slightly hygroscopic.

### Biological assay data

### Assay example 1 Assay of the activity of the compounds of the present disclosure on human gonadotropin-releasing hormone receptor

Assay purpose: FLIPR assay technology was used to assay the inhibitory activity of a test compound on gonadotropin-releasing hormone receptor at cellular level
Main assay materials and sources:
Fluo-4 Direct^{™} Kit - Invitrogen-F10471
Poly-L-lysine coated 384-well cell plate - Greiner-781946
384-well compound plate - Greiner-781280
ECHO (Acoustic Liquid Handling System) for compound preparation - Labcyte
FLIPR (Fluorometric Imaging Plate Reader) - Molecular Devices
Assay procedure:
   GnRH/HEK293 (human embryonic kidney cell 293) cultured cells in the logarithmic growth phase were washed with DPBS (Dulbecco's phosphate buffered saline) buffer, and an appropriate amount of 0.05% EDTA (ethylenediaminetetraacetic acid)-trypsin was added. The cells were placed in a carbon dioxide incubator at 37 °C for digestion for 1-2 minutes, and then removed from the incubator. A medium was added to the cells to terminate the digestion. Cells were dispersed by repeated pipetting and harvested by centrifugation. The cells were seeded into a 384-well Poly-Lysine-Coated Cell Plate at a density of 20,000 cells per well, 20 µL, and incubated overnight in a 5% CO₂, 37°C incubator.

On the next day, 20 µL of 2×Fluo-4 Direct^{™} buffer was added to each well, and the plate was incubated in a 5% CO₂, 37°C incubator for 50 minutes. The cells were placed at room temperature for 10 minutes. 0.2 mM Leuprolide acetate was diluted to 10 concentrations by 4-fold serial dilution in ECHO, and 900 nL of that was transferred to the compound plate. 30 µL of FLIPR buffered saline was added to the compound plate, and the plate was centrifuged at 1000 rpm for 1 min. The software of the FLIPR instrument was run, and 10 µL of assay buffer salt solution was added according to the set program. The fluorescence signal was read. Then 10 µL of the reference compound as an agonist was added, and the fluorescent signal was read. EC₈₀ was calculated, and the agonist with a concentration of 6×EC₈₀ was prepared.

2 mM assay compounds and an appropriate concentration of reference compound were serially diluted 4-fold to 10 concentrations in ECHO, and 900 nL of that was transferred to a compound plate. 30 µL of FLIPR buffered saline was added to the compound plate, and the plate was centrifuged at 1000 rpm for 1 min. The software of the FLIPR instrument was run, and 10 µL of assay and reference compounds were added to the cell plate according to the set program. The fluorescent signal was read. Then 10 µL of agonist with a concentration of 6×EC₈₀ was added to the cell plate and the fluorescent signal was read.

IC₅₀ of the compound to inhibit the calcium flow of gonadotropin-releasing hormone receptor, i.e., the drug concentration when the Ca²⁺ flow was inhibited by half in the cells stably expressing the GnRH receptor, was calculated. The IC₅₀ of the drug was calculated by GraphPad Prism 5.0 software.

### Assay results:

The inhibitory activity of the compound of the present disclosure on human gonadotropin-releasing hormone receptor was determined by the above assay method, and the measured IC₅₀ is shown in Table 26:

**Table 26 IC₅₀ of the compound of the present disclosure for inhibiting the activity of human gonadotropin-releasing hormone receptor**

| Compound No. | IC₅₀ (nM) |
|---|---|
| Compound of formula (I) | 3.2 |

Conclusion: The compound of the present disclosure has a significant inhibitory effect on human gonadotropin-releasing hormone receptor.

### Assay example 2 Pharmacokinetic evaluation

Assay purpose: The pharmacokinetic properties of the compounds of the present disclosure were studied in mice.

### Assay protocol:

Each assay compound was mixed with DMAC respectively, and vortexed for 2 minutes. The solution of the assay compound in DMAC was mixed and vortexed for 2 minutes to prepare a clear solution of 10 mg/mL. 0.0600 mL of 10 mg/mL solution was added to 0.300 mL of Solutol, and the mixture was vortexed for 2 minutes. Then 2.400 mL of normal saline was added, and the mixture was vortexed for 2 minutes to obtain a clear solution of 0.2 mg/mL, which was used for administration in PO group. 0.500 mL of the solution for administration in PO group was vortexed for 2 minutes, and 0.0500 mL of DMAC was added. The mixture was vortexed for 2 minutes, and then 0.0500 mL of solutol was added. The mixture was vortexed for 2 minutes, and finally 0.400 mL of normal saline was added. The mixture was vortexed for two minutes to give a clear solution of 0.1 mg/mL, which was filtered through a microporous membrane to give a solution for administration in injection (IV) group.

Four male CD-1 mice were divided into two groups. The animals in the first group were administered intravenously once at a dose of 0.5 mg/kg, wherein the vehicle was 10% DMAC/10% Solutol/80% normal saline, and the administration volume was 5 mL/kg. The animals in the second group were administered orally 2 mg/kg of the assay compound by oral gavage, wherein the oral vehicle was 10% DMAC/10% Solutol/80% normal saline, and the oral volume was 10 mL/kg. Whole blood was collected at 0.033 (IV injection only), 0.083, 0.25, 0.5, 1, 2, 4 and 12 hours post-dose. The whole blood was centrifuged at 3200g at 2-8°C for 10 min to obtain plasma. The concentration of the assay compound in plasma was determined by LC/MS/MS method, and the pharmacokinetic parameters were calculated by Phoenix WinNonlin software.

### Assay results:

The assay results are shown in Table 27. The parameters have the following meanings: IV: intravenous injection; PO: oral administration; C₀: initial plasma concentration; Cₘₐₓ: maximum drug concentration in the systemic circulation; Tₘₐₓ: time required to reach Cₘₐₓ; T_{1/2}: half-life; V_{dss}: apparent volume of distribution; Cl: clearance rate; AUC₀₋ₗₐₛₜ: area under the plasma drug concentration-time curve.

**Table 27 Pharmacokinetic (PK) assay results of the compound of formula (I) in plasma**

| PK parameter | IV (0.5 mg/kg) | PO (2 mg/kg) |
|---|---|---|
| C₀ (nmol/L) | 13513 | -- |
| Cₘₐₓ (nmol/L) | -- | 25800 |
| Tₘₐₓ (h) | -- | 0.500 |
| T_{1/2} (h) | 5.66 | 5.03 |
| V_{dss} (L/kg) | 0.159 | -- |
| Cl (mL/min/kg) | 0.332 | -- |
| AUC₀₋ₗₐₛₜ (h*nmol/L) | 48307 | 142824 |
| Bioavailability (%) | -- | 95.3 |

| | | |
|---|---|---|
| "--" means not tested or no data available. | | |

Conclusion: The compound of the present disclosure has high exposure in plasma, low clearance rate, long half-life, and high oral bioavailability, and exhibits excellent pharmacokinetic properties.

## Claims

1. A crystal form B of the compound of formula (I), 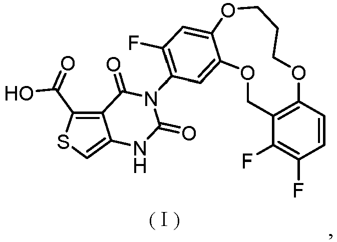 which is **characterized by** an X-ray powder diffraction pattern obtained using Cu Kα radiation with characteristic diffraction peaks at 2θ angles of 4.62±0.20°, 7.35±0.20°, and 18.34±0.20°.

2. The crystal form B according to claim 1, which is **characterized by** an X-ray powder diffraction pattern with characteristic diffraction peaks at 2θ angles of 4.62±0.20°, 7.35±0.20°, 11.45±0.20°, 12.33±0.20°, 18.34±0.20°, 22.41±0.20°, 26.54±0.20°, and 27.08±0.20°.

3. The crystal form B according to claim 2, which is **characterized by** an X-ray powder diffraction pattern with characteristic diffraction peaks at 2θ angles of 4.62±0.20°, 7.35±0.20°, 11.45±0.20°, 12.33±0.20°, 13.75±0.20°, 17.89±0.20°, 18.34±0.20°, 20.92±0.20°, 22.41±0.20°, 25.43±0.20°, 26.54±0.20°, and 27.08±0.20°.

4. The crystal form B according to claim 3, which is **characterized by** an X-ray powder diffraction pattern with characteristic diffraction peaks at 2θ angles of 4.62±0.20°, 7.35±0.20°, 9.17±0.20°, 11.45±0.20°, 12.33±0.20°, 13.75±0.20°, 17.89±0.20°, 18.34±0.20°, 20.92±0.20°, 22.41±0.20°, 23.57±0.20°, 24.46±0.20°, 25.43±0.20°, 26.54±0.20°, 27.08±0.20°, and 28.81±0.20°.

5. The crystal form B according to claim 4, which is **characterized by** an X-ray powder diffraction pattern with characteristic diffraction peaks at 2θ angles of 4.62°, 7.35°, 9.17°, 11.45°, 12.33°, 13.17°, 13.75°, 14.34°, 14.67°, 16.50°, 17.43°, 17.89°, 18.34°, 19.45°, 20.92°, 21.68°, 22.41°, 23.57°, 24.46°, 25.43°, 25.89°, 26.54°, 27.08°, and 28.81°.

6. The crystal form B according to any one of claims 1 to 5, which is **characterized by** it has any one of the following characteristics:
(1) the XRPD pattern thereof is substantially as shown in Fig. 1;
(2) the differential scanning calorimetry curve thereof has a peak value of an exothermic peak at 224.4 °C±3 °C;
(3) the DSC curve thereof is substantially as shown in Fig. 2;
(4) the thermogravimetric analysis curve thereof has a weight loss of 1.12% at 150 °C±3 °C;
(5) the TGA curve thereof is substantially as shown in Fig. 3.

7. A crystal form C of the compound of formula (I), 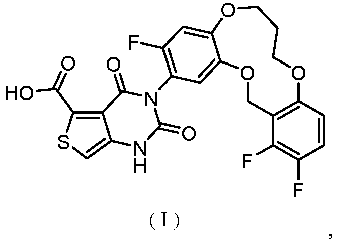 which is **characterized by** an X-ray powder diffraction pattern obtained using Cu Kα radiation with characteristic diffraction peaks at any of the following groups of 2θ angles:
(1) 7.18±0.20°, 8.47±0.20°, and 12.80±0.20°;
(2) 7.18±0.20°, 8.47±0.20°, 12.80±0.20°, 15.80±0.20°, 16.96±0.20°, 19.23±0.20°, 20.12±0.20°, and 23.30±0.20°;
(3) 7.18±0.20°, 8.47±0.20°, 11.55±0.20°, 12.80±0.20°, 15.80±0.20°, 16.96±0.20°, 19.23±0.20°, 20.12±0.20°, 23.30±0.20°, and 26.31±0.20°;
(4) 7.18°, 8.47°, 11.55°, 12.80°, 15.80°, 16.96°, 19.23°, 20.12°, 23.30°, and 26.31°.

8. The crystal form C according to claim 7, which is **characterized by** it has any one of the following characteristics:
(1) the XRPD pattern thereof is substantially as shown in Fig. 4;
(2) the differential scanning calorimetry curve thereof has a peak value of an exothermic peak at 225.1 °C±3 °C;
(3) the DSC curve thereof is substantially as shown in Fig. 5;
(4) the thermogravimetric analysis curve thereof has a weight loss of 1.13% at 150 °C±3 °C;
(5) the TGA curve thereof is substantially as shown in Fig. 6.

9. A pharmaceutically acceptable salt of the compound of formula (I), 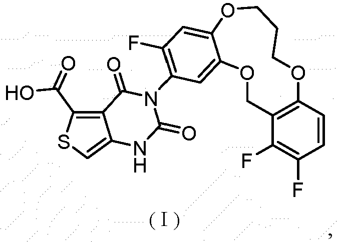 wherein, the pharmaceutically acceptable salt is a lysine salt, a dibenzylethylenediamine salt, a choline salt, a meglumine salt, a triethylamine salt, an aluminum salt, a zinc salt, or a lithium salt.

10. The pharmaceutically acceptable salt of the compound of formula (I) according to claim 9, wherein, the structure of the choline salt of the compound of formula (I) is shown in formula (II), and the structure of the dibenzylethylenediamine salt of the compound of formula (I) is shown in formula (IV), 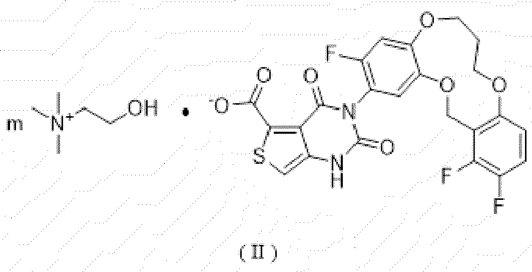 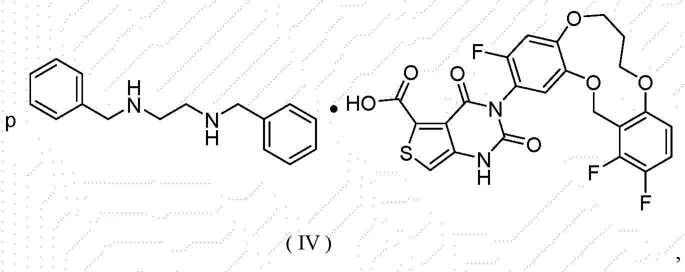 wherein, m is selected from 0.5 to 1.5; and p is selected from 0.4 to 1.5.

11. The pharmaceutically acceptable salt of the compound of formula (I) according to claim 10, wherein, the compound of formula (II) is a compound of formula (II-1),

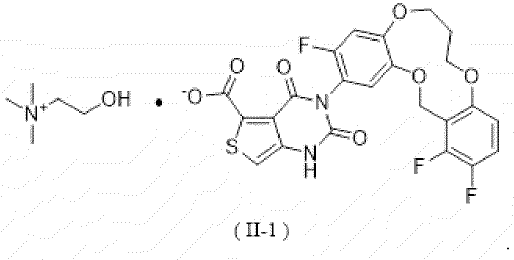

12. A crystal form S1 of the compound of formula (II-1), 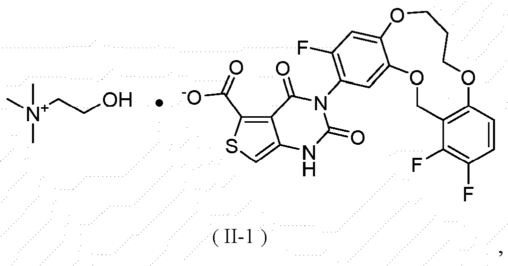 **which is characterized by** an X-ray powder diffraction pattern obtained using Cu Kα radiation with characteristic diffraction peaks at any of the following groups of 2θ angles:
(1) 8.68±0.20°, 12.70±0.20°, 18.12±0.20°, 19.43±0.20°, and 24.41±0.20°;
(2) 8.68±0.20°, 12.27±0.20°, 12.70±0.20°, 15.20±0.20°, 18.12±0.20°, 18.95±0.20°, 19.43±0.20°, and 24.41±0.20°;
(3) 8.68±0.20°, 12.27±0.20°, 12.70±0.20°, 15.20±0.20°, 16.94±0.20°, 17.37±0.20°, 18.12±0.20°, 18.95±0.20°, 19.43±0.20°, 24.41±0.20°, 25.51±0.20°, and 27.62±0.20°;
(4) 5.98°, 8.68°, 9.67°, 11.39°, 12.27°, 12.70°, 13.61°, 15.20°, 16.09°, 16.94°, 17.37°, 18.12°, 18.95°, 19.43°, 19.93°, 20.35°, 20.91°, 21.44°, 21.69°, 22.05°, 23.32°, 23.85°, 24.41°, 25.51°, 27.11°, 27.62°, 29.05°, and 29.85°.

13. The crystal form S1 according to claim 12, wherein the XRPD pattern thereof is substantially as shown in Fig. 19.

14. The crystal form B according to any one of claims 1 to 6, the crystal form C according to claim 7 or 8, the crystal form S1 according to claim 12 or 13, or the salt according to any one of claims 9 to 11, for use in the treatment of a GnRH receptor-related disease.

15. The crystal form B according to any one of claims 1 to 6, the crystal form C according to claim 7 or 8, the crystal form S1 according to claim 12 or 13, or the salt according to any one of claims 9 to 11, for use in the prevention and/or treatment of an endometriosis and/or uterine fibroid-related disease.

## Patentansprüche

1. Kristallform B der Verbindung der Formel (I), 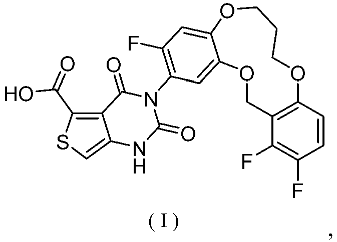 die durch ein unter Verwendung von Cu-Kα-Strahlung erhaltenes Röntgenpulverdiffraktogramm mit charakteristischen Beugungspeaks bei 2θ-Winkeln von 4,62±0,20°, 7,35±0,20° und 18,34±0,20° gekennzeichnet ist.

2. Kristallform B nach Anspruch 1, die durch ein Röntgenpulverdiffraktogramm mit charakteristischen Beugungspeaks bei 2θ-Winkeln von 4,62±0,20°, 7,35±0,20°, 11,45±0,20°, 12,33±0,20°, 18,34±0,20°, 22,41±0,20°, 26,54±0,20° und 27,08±0,20° gekennzeichnet ist.

3. Kristallform B nach Anspruch 2, die durch ein Röntgenpulverdiffraktogramm mit charakteristischen Beugungspeaks bei 2θ-Winkeln von 4,62±0,20°, 7,35±0,20°, 11,45±0,20°, 12,33±0,20°, 13,75±0,20°, 17,89±0,20°, 18,34±0,20°, 20,92±0,20°, 22,41±0,20°, 25,43±0,20°, 26,54±0,20° und 27,08±0,20° gekennzeichnet ist.

4. Kristallform B nach Anspruch 3, die durch ein Röntgenpulverdiffraktogramm mit charakteristischen Beugungspeaks bei 2θ-Winkeln von 4,62±0,20°, 7,35±0,20°, 9,17±0,20°, 11,45±0,20°, 12,33±0,20°, 13,75±0,20°, 17,89±0,20°, 18,34±0,20°, 20,92±0,20°, 22,41±0,20°, 23,57±0,20°, 24,46±0,20°, 25,43±0,20°, 26,54±0,20°, 27,08±0,20° und 28,81±0,20° gekennzeichnet ist.

5. Kristallform B nach Anspruch 4, die durch ein Röntgenpulverdiffraktogramm mit charakteristischen Beugungspeaks bei 2θ-Winkeln von 4,62°, 7,35°, 9,17°, 11,45°, 12,33°, 13,17°, 13,75°, 14,34°, 14,67°, 16,50°, 17,43°, 17,89°, 18,34°, 19,45°, 20,92°, 21,68°, 22,41°, 23,57°, 24,46°, 25,43°, 25,89°, 26,54°, 27,08° und 28,81° gekennzeichnet ist.

6. Kristallform B nach einem der Ansprüche 1 bis 5, die **dadurch gekennzeichnet ist, dass** sie eines der folgenden Merkmale aufweist:
(1) ihr XRPD-Diffraktogramm ist im Wesentlichen wie in Fig. 1 dargestellt;
(2) ihre Kurve der dynamischen Differenzkalorimetrie weist einen Peakwert eines exothermen Peaks bei 224,4 °C±3 °C auf;
(3) ihre DSC-Kurve ist im Wesentlichen wie in Fig. 2 dargestellt;
(4) ihre Kurve der thermogravimetrischen Analyse weist bei 150 °C±3 °C einen Gewichtsverlust von 1,12 % auf;
(5) ihre TGA-Kurve ist im Wesentlichen wie in Fig. 3 dargestellt.

7. Kristallform C der Verbindung der Formel (I), 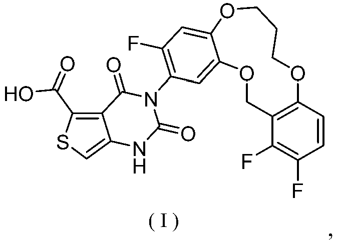 die durch ein unter Verwendung von Cu-Kα-Strahlung erhaltenes Röntgenpulverdiffraktogramm mit charakteristischen Beugungspeaks bei einer der folgenden Gruppen von 2θ-Winkeln gekennzeichnet ist:
(1) 7,18±0,20°, 8,47±0,20° und 12,80±0,20°;
(2) 7,18±0,20°, 8,47±0,20°, 12,80±0,20°, 15,80±0,20°, 16,96±0,20°, 19,23±0,20°, 20,12±0,20° und 23,30±0,20°;
(3) 7,18±0,20°, 8,47±0,20°, 11,55±0,20°, 12,80±0,20°, 15,80±0,20°, 16,96±0,20°, 19,23±0,20°, 20,12±0,20°, 23,30±0,20° und 26,31=+0,20°;
(4) 7,18°, 8,47°, 11,55°, 12,80°, 15,80°, 16,96°, 19,23°, 20,12°, 23,30° und 26,31°.

8. Kristallform C nach Anspruch 7, die **dadurch gekennzeichnet ist, dass** sie eines der folgenden Merkmale aufweist:
(1) ihr XRPD-Diffraktogramm ist im Wesentlichen wie in Fig. 4 dargestellt;
(2) ihre Kurve der dynamischen Differenzkalorimetrie weist einen Peakwert eines exothermen Peaks bei 225,1 °C±3 °C auf;
(3) ihre DSC-Kurve ist im Wesentlichen wie in Fig. 5 dargestellt;
(4) ihre Kurve der thermogravimetrischen Analyse weist bei 150 °C±3 °C einen Gewichtsverlust von 1,13 % auf;
(5) ihre TGA-Kurve ist im Wesentlichen wie in Fig. 6 dargestellt.

9. Pharmazeutisch verträgliches Salz der Verbindung der Formel (I), 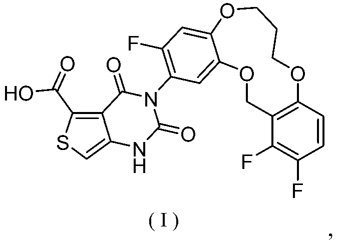 wobei das pharmazeutisch verträgliche Salz ein Lysinsalz, ein Dibenzylethylendiaminsalz, ein Cholinsalz, ein Megluminsalz, ein Triethylaminsalz, ein Aluminiumsalz, ein Zinksalz oder ein Lithiumsalz ist.

10. Pharmazeutisch verträgliches Salz der Verbindung der Formel (I) nach Anspruch 9, wobei die Struktur des Cholinsalzes der Verbindung der Formel (I) in Formel (II) dargestellt ist und die Struktur des Dibenzylethylendiaminsalzes der Verbindung der Formel (I) in Formel (IV) dargestellt ist, 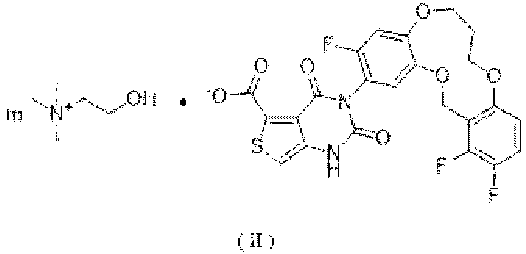 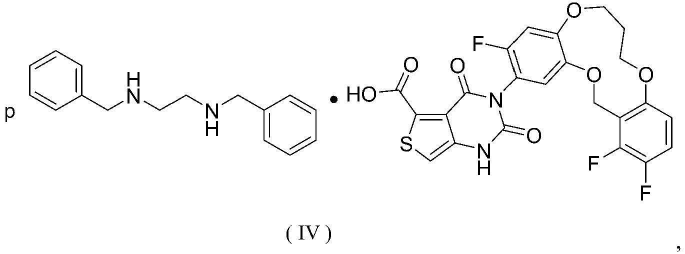 wobei m aus 0,5 bis 1,5 ausgewählt ist und p aus 0,4 bis 1,5 ausgewählt ist.

11. Pharmazeutisch verträgliches Salz der Verbindung der Formel (I) nach Anspruch 10, wobei die Verbindung der Formel (II) eine Verbindung der Formel (II-1) ist,

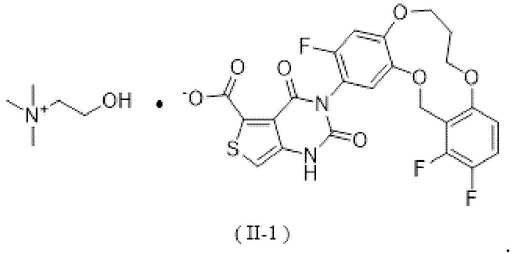

12. Kristallform S1 der Verbindung der Formel (II-1), 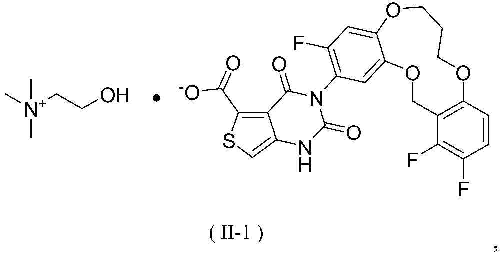 die durch ein unter Verwendung von Cu-Kα-Strahlung erhaltenes Röntgenpulverdiffraktogramm mit charakteristischen Beugungspeaks bei einer der folgenden Gruppen von 2θ-Winkeln gekennzeichnet ist:
(1) 8,68±0,20°, 12,70±0,20°, 18,12±0,20°, 19,43±0,20° und 24,41±0,20°;
(2) 8,68±0,20°, 12,27±0,20°, 12,70±0,20°, 15,20±0,20°, 18,12±0,20°, 18,95±0,20°, 19,43±0,20° und 24,41±0,20°;
(3) 8,68±0,20°, 12,27±0,20°, 12,70±0,20°, 15,20±0,20°, 16,94±0,20°, 17,37±0,20°, 18,12±0,20°, 18,95±0,20°, 19,43±0,20°, 24,41±0,20°, 25,51±0,20° und 27,62±0,20°;
(4) 5,98°, 8,68°, 9,67°, 11,39°, 12,27°, 12,70°, 13,61°, 15,20°, 16,09°, 16,94°, 17,37°, 18,12°, 18,95°, 19,43°, 19,93°, 20,35°, 20,91°, 21,44°, 21,69°, 22,05°, 23,32°, 23,85°, 24,41°, 25,51°, 27,11°, 27,62°, 29,05° und 29,85°.

13. Kristallform S1 nach Anspruch 12, wobei ihr XRPD-Diffraktogramm im Wesentlichen wie in Fig. 19 dargestellt ist.

14. Kristallform B nach einem der Ansprüche 1 bis 6, Kristallform C nach Anspruch 7 oder 8, Kristallform S1 nach Anspruch 12 oder 13 oder Salz nach einem der Ansprüche 9 bis 11 zur Verwendung bei der Behandlung einer mit dem GnRH-Rezeptor in Zusammenhang stehenden Erkrankung.

15. Kristallform B nach einem der Ansprüche 1 bis 6, Kristallform C nach Anspruch 7 oder 8, Kristallform S1 nach Anspruch 12 oder 13 oder Salz nach einem der Ansprüche 9 bis 11 zur Verwendung bei der Prävention und/oder Behandlung einer mit Endometriose und/oder Uterusmyomen in Zusammenhang stehenden Erkrankung.

## Revendications

1. Forme cristalline B du composé de formule (I), 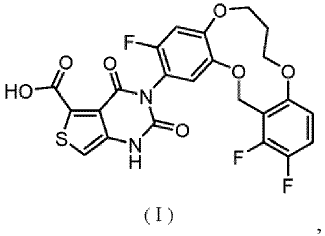 qui se **caractérise par** un diagramme de diffraction de rayons X sur poudre obtenu à l'aide d'un rayonnement Cu Kα avec des pics de diffraction caractéristiques à des angles 20 de 4,62±0,20°, 7,35±0,20° et 18,34±0,20°.

2. Forme cristalline B selon la revendication 1, qui se **caractérise par** un diagramme de diffraction de rayons X sur poudre avec des pics de diffraction caractéristiques à des angles 2θ de 4,62±0,20°, 7,35±0,20°, 11,45±0,20°, 12,33±0,20°, 18,34±0,20°, 22,41±0,20°, 26,54±0,20°, et 27,08±0,20°.

3. Forme cristalline B selon la revendication 2, qui se **caractérise par** un diagramme de diffraction de rayons X sur poudre avec des pics de diffraction caractéristiques à des angles 2θ de 4,62±0,20°, 7,35±0,20°, 11,45±0,20°, 12,33±0,20°, 13,75±0,20°, 17,89±0,20°, 18,34±0,20°, 20,92±0,20°, 22,41±0,20°, 25,43±0,20°, 26,54±0,20°, et 27,08±0,20°.

4. Forme cristalline B selon la revendication 3, qui se **caractérise par** un diagramme de diffraction de rayons X sur poudre avec des pics de diffraction caractéristiques à des angles 20 de 4,62±0,20°, 7,35±0,20°, 9,17±0,20°, 11,45±0,20°, 12,33±0,20°, 13,75±0,20°, 17,89±0,20°, 18,34±0,20°, 20,92±0,20°, 22,41±0,20°, 23,57±0,20°, 24,46±0,20°, 25,43±0,20°, 26,54±0,20°, 27,08±0,20°, et 28,81±0,20°.

5. Forme cristalline B selon la revendication 4, qui se **caractérise par** un diagramme de diffraction de rayons X sur poudre avec des pics de diffraction caractéristiques à des angles 2θ de 4,62°, 7,35°, 9,17°, 11,45°, 12,33°, 13,17°, 13,75°, 14,34°, 14,67°, 16,50°, 17,43°, 17,89°, 18,34°, 19,45°, 20,92°, 21,68°, 22,41°, 23,57°, 24,46°, 25,43°, 25,89°, 26,54°, 27,08°, et 28,81°.

6. Forme cristalline B selon l'une des revendications 1 à 5, qui se **caractérise par** l'une des caractéristiques suivantes :
(1) le diagramme XRPD de celle-ci est sensiblement tel que montré sur la figure 1 ;
(2) la courbe de calorimétrie différentielle à balayage de celle-ci présente une valeur de crête d'un pic exothermique à 224,4 °C±3 °C ;
(3) la courbe DSC de celle-ci est sensiblement telle que montrée sur la figure 2 ;
(4) la courbe d'analyse thermogravimétrique de celle-ci présente une perte pondérale de 1,12 % à 150 °C±3 °C ;
(5) la courbe ATG de celle-ci est sensiblement telle que montrée sur la figure 3.

7. Forme cristalline C du composé de formule (I), 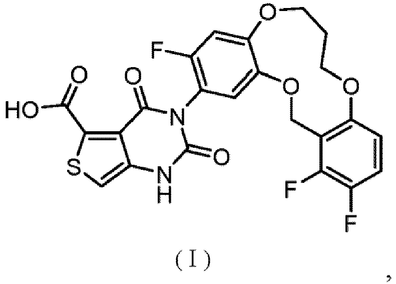 qui se **caractérise par** un diagramme de diffraction de rayons X sur poudre obtenu à l'aide d'un rayonnement Cu Kα avec des pics de diffraction caractéristiques au niveau de l'un quelconque des groupes suivants d'angles 2θ :
(1) 7,18±0,20°, 8,47±0,20° et 12,80±0,20° ;
(2) 7,18±0,20°, 8,47±0,20°, 12,80±0,20°, 15,80±0,20°, 16,96±0,20°, 19,23±0,20°, 20,12±0,20° et 23,30±0,20° ;
(3) 7,18±0,20°, 8,47±0,20°, 11,55±0,20°, 12,80±0,20°, 15,80±0,20°, 16,96±0,20°, 19,23±0,20°, 20,12±0,20°, 23,30±0,20° et 26,31±0,20° ;
(4) 7,18°, 8,47°, 11,55°, 12,80°, 15,80°, 16,96°, 19,23°, 20,12°, 23,30° et 26,31°.

8. Forme cristalline C selon la revendication 7, qui se **caractérise en ce qu'**elle présente l'une quelconque des caractéristiques suivantes :
(1) le diagramme XRPD de celle-ci est sensiblement tel que montré sur la figure 4 ;
(2) la courbe de calorimétrie différentielle à balayage de celle-ci présente une valeur de crête d'un pic exothermique à 225,1 °C±3 °C ;
(3) la courbe DSC de celle-ci est sensiblement telle que montrée sur la figure 5 ;
(4) la courbe d'analyse thermogravimétrique de celle-ci présente une perte pondérale de 1,13 % à 150 °C±3 °C ;
(5) la courbe ATG de celle-ci est sensiblement telle que montrée sur la figure 6.

9. Sel pharmaceutiquement acceptable du composé de formule (I), 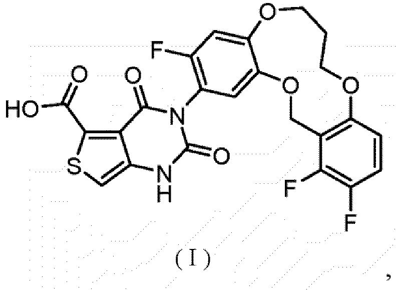 dans lequel le sel pharmaceutiquement acceptable est un sel de lysine, un sel de dibenzyléthylènediamine, un sel de choline, un sel de méglumine, un sel de triéthylamine, un sel d'aluminium, un sel de zinc ou un sel de lithium.

10. Sel pharmaceutiquement acceptable du composé de formule (I) selon la revendication 9, dans lequel la structure du sel de choline du composé de formule (I) est montrée dans la formule (II), et la structure du sel de dibenzyléthylènediamine du composé de formule (I) est montrée dans la formule (IV), 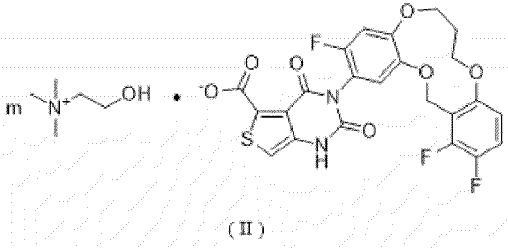 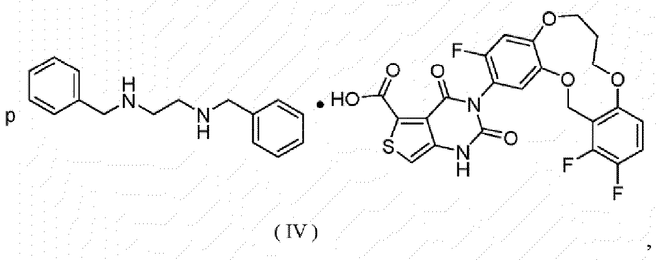 dans lequel m est sélectionné de 0,5 à 1,5 ; et p est sélectionné de 0,4 à 1,5.

11. Sel pharmaceutiquement acceptable du composé de formule (I) selon la revendication 10, dans lequel le composé de formule (II) est un composé de formule (II-1),

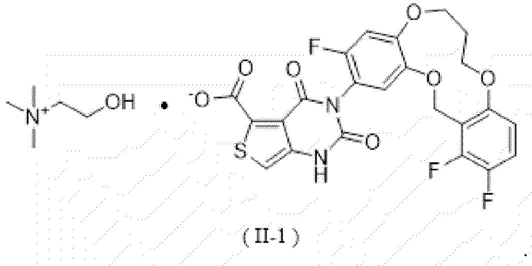

12. Forme cristalline S1 du composé de formule (II-1), 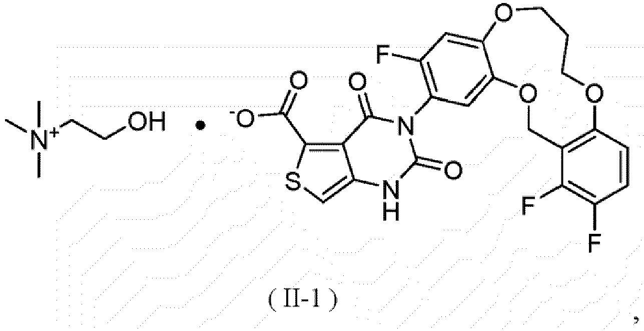 qui se **caractérise par** un diagramme de diffraction de rayons X sur poudre obtenu à l'aide d'un rayonnement Cu Kα avec des pics de diffraction caractéristiques au niveau de l'un quelconque des groupes suivants d'angles 20 :
(1) 8,68±0,20°, 12,70±0,20°, 18,12±0,20°, 19,43±0,20° et 24,41±0,20° ;
(2) 8,68±0,20°, 12,27±0,20°, 12,70±0,20°, 15,20±0,20°, 18,12±0,20°, 18,95±0,20°, 19,43±0,20° et 24,41±0,20° ;
(3) 8,68±0,20°, 12,27±0,20°, 12,70±0,20°, 15,20±0,20°, 16,94±0,20°, 17,37±0,20°, 18,12±0,20°, 18,95±0,20°, 19,43±0,20°, 24,41±0,20°, 25,51±0,20° et 27,62±0,20° ;
(4) 5,98°, 8,68°, 9,67°, 11,39°, 12,27°, 12,70°, 13,61°, 15,20°, 16,09°, 16,94°, 17,37°, 18,12°, 18,95°, 19,43°, 19,93°, 20,35°, 20,91°, 21,44°, 21,69°, 22,05°, 23,32°, 23,85°, 24,41°, 25,51°, 27,11°, 27,62°, 29,05° et 29,85°.

13. Forme cristalline S1 selon la revendication 12, dans laquelle le diagramme XRPD de celle-ci est sensiblement tel que montré sur la figure 19.

14. Forme cristalline B selon l'une des revendications 1 à 6, forme cristalline C selon la revendication 7 ou 8, forme cristalline S1 selon la revendication 12 ou 13, ou sel selon l'une des revendications 9 à 11, pour une utilisation dans le traitement d'une maladie liée au récepteur de la GnRH.

15. Forme cristalline B selon l'une des revendications 1 à 6, forme cristalline C selon la revendication 7 ou 8, forme cristalline S1 selon la revendication 12 ou 13, ou sel selon l'une des revendications 9 à 11, pour une utilisation dans la prévention et/ou le traitement d'une maladie liée à une endométriose et/ou à un fibrome utérin.
